# FASCICULE DE BREVET EUROPEEN

(11) **EP 3 027 731 B1**
(45) Date de publication et mention de la délivrance du brevet: **14.10.2020**
(21) Numéro de dépôt: 14747616.2
(22) Date de dépôt: 31.07.2014
(51) Int. Cl.: C12N 1/00, G01N 33/68, C12N 5/071, C12N 5/02

(54) **REACTIF DE STIMULATION, SUBSTITUT CUTANE, EXPLANT CUTANE ET PROCEDE POUR REPRODUIRE LA PHYSIOPATHOLOGIE DE LA DERMATITE ATOPIQUE**
IL-1-BETA ZUR PROINFLAMMATORISCHEN STIMULATION EINES HAUTERSATZES ODER EXPLANTATS
IL-1 BETA FOR THE PRO-INFLAMMATORY STIMULATION OF A CUTANEOUS SUBSTITUTE OR EXPLANT

(30) Priorité: 31.07.2013 FR 1357608; 28.11.2013 FR 1361788
(43) Date de publication de la demande: 08.06.2016
(73) Titulaire: Pierre Fabre Dermo-Cosmétique, 92100 Boulogne-Billancourt (FR)
(72) Inventeur: GALLIANO, Marie-Florence, F-31700 Blagnac (FR); BERNARD, Marine, F-82100 Castelsarrasin (FR)
(74) Mandataire: Regimbeau
(86) Numéro de dépôt international: PCT/EP2014/066518
(87) Numéro de publication internationale: WO 2015/014949

(56) Documents cités:
- WO-A1-2008/110569
- WO-A1-2008/110569
- WO-A1-2010/049152
- WO-A1-2010/049152
- WO-A2-2009/120891
- WO-A2-2009/120891
- US-A1- 2005 003 533
- US-A1- 2005 003 533
- J. PENA ET AL: "Pseudomonas aeruginosa Inhibition of Flagellin-Activated NF-?B and Interleukin-8 by Human Airway Epithelial Cells", INFECTION AND IMMUNITY, vol. 77, no. 7, 18 mai 2009 (2009-05-18), pages 2857-2865, XP055126005, ISSN: 0019-9567, DOI: 10.1128/IAI.01355-08
- H. S. HREGGVIDSDOTTIR ET AL: "The alarmin HMGB1 acts in synergy with endogenous and exogenous danger signals to promote inflammation", JOURNAL OF LEUKOCYTE BIOLOGY, vol. 86, no. 3, 29 juin 2009 (2009-06-29), pages 655-662, XP055126006, ISSN: 0741-5400, DOI: 10.1189/jlb.0908548
- MACFARLANE T V ET AL: "PS1-46 Amnion epithelial cells are a source of thymic stromal lymphopoietin", CYTOKINE, ACADEMIC PRESS LTD, PHILADELPHIA, PA, US, vol. 52, no. 1-2, 1 octobre 2010 (2010-10-01), pages 26-27, XP027261695, ISSN: 1043-4666 [extrait le 2010-09-02]
- SOUMELIS V ET AL: "Human epithelial cells trigger dendritic cell mediated allergic inflammation by producing TSLP", NATURE IMMUNOLOGY, NATURE PUBLISHING GROUP US, NEW YORK, vol. 3, no. 7, 1 juillet 2002 (2002-07-01) , pages 673-680, XP002364946, ISSN: 1529-2908
- STEVEN F ZIEGLER ET AL: "Sensing the outside world: TSLP regulates barrier immunity", NATURE IMMUNOLOGY, vol. 11, no. 4, 19 mars 2010 (2010-03-19), pages 289-293, XP055453580, New York ISSN: 1529-2908, DOI: 10.1038/ni.1852
- S. I. BOGIATZI ET AL: "Cutting Edge: Proinflammatory and Th2 Cytokines Synergize to Induce Thymic Stromal Lymphopoietin Production by Human Skin Keratinocytes", THE JOURNAL OF IMMUNOLOGY, vol. 178, no. 6, 15 mars 2007 (2007-03-15) , pages 3373-3377, XP055146979, ISSN: 0022-1767, DOI: 10.4049/jimmunol.178.6.3373
- YANG XIE ET AL: "Longtranscript expression and release of TSLP induced by TLR ligands and cytokines in human keratinocytes", JOURNAL OF DERMATOLOGICAL SCIENCE, ELSEVIER, AMSTERDAM, NL, vol. 66, no. 3, 22 mars 2012 (2012-03-22), pages 233-237, XP028507069, ISSN: 0923-1811, DOI: 10.1016/J.JDERMSCI.2012.03.007 [extrait le 2012-03-30]
- RYUTA KAMEKURA ET AL: "Thymic stromal lymphopoietin enhances tight-junction barrier function of human nasal epithelial cells", CELL AND TISSUE RESEARCH, SPRINGER, BERLIN, DE, vol. 338, no. 2, 9 septembre 2009 (2009-09-09), pages 283-293, XP019760811, ISSN: 1432-0878, DOI: 10.1007/S00441-009-0855-1
- SONIA SCHUEPBACH-MALLEPELL ET AL: "Antagonistic effect of the inflammasome on thymic stromal lymphopoietin expression in the skin", JOURNAL OF ALLERGY AND CLINICAL IMMUNOLOGY, vol. 132, no. 6, 13 août 2013 (2013-08-13) , pages 1348-1357, XP055146999, ISSN: 0091-6749, DOI: 10.1016/j.jaci.2013.06.033
- ALLAKHVERDI ZOULFIA ET AL: "Thymic stfomal lymphopoietin is released by human epithelial cells in response to microbes, trauma, or inflammation and potently activates mast cells", THE JOURNAL OF EXPERIMENTAL MEDICINE, ROCKEFELLER UNIVERSITY PRESS, US, vol. 204, no. 2, 1 février 2007 (2007-02-01), pages 253-258, XP002511407, ISSN: 0022-1007, DOI: 10.1084/JEM.20062211
- DATABASE BIOSIS [Online] BIOSCIENCES INFORMATION SERVICE, PHILADELPHIA, PA, US; mai 2011 (2011-05), COOK ELLEN B ET AL: "Human Conjunctival Epithelial Cells Express the Receptor for TSLP", XP002731228, Database accession no. PREV201200524586 & ARVO ANNUAL MEETING ABSTRACT SEARCH AND PROGRAM PLANNER, vol. 2011, mai 2011 (2011-05), page 1120, ANNUAL MEETING OF THE ASSOCIATION-FOR-RESEARCH-IN-VISION-AND-OPH THALMOLOGY (ARVO); FT LAUDERDALE, FL, USA; MAY 01 -05, 2011
- LEE HAI-CHON ET AL: "Inducible expression of the proallergic cytokine thymic stromal lymphopoietin in airway epithelial cells is controlled by NF kappa B", PROCEEDINGS OF THE NATIONAL ACADEMY OF SCIENCES, NATIONAL ACADEMY OF SCIENCES, vol. 104, no. 3, 16 janvier 2007 (2007-01-16), pages 914-919, XP002472961, ISSN: 0027-8424, DOI: 10.1073/PNAS.0607305104
- JENSEN SIMON S ET AL: "Differential induction of inflammatory cytokines by dendritic cells treated with novel TLR-agonist and cytokine based cocktails: targeting dendritic cells in autoimmunity", JOURNAL OF INFLAMMATION, BIOMED CENTRAL, LONDON, GB, vol. 7, no. 1, 27 juillet 2010 (2010-07-27), page 37, XP021078249, ISSN: 1476-9255, DOI: 10.1186/1476-9255-7-37
- J. PENA ET AL: "Pseudomonas aeruginosa Inhibition of Flagellin-Activated NF-?B and Interleukin-8 by Human Airway Epithelial Cells", INFECTION AND IMMUNITY, vol. 77, no. 7, 18 May 2009 (2009-05-18), pages 2857-2865, XP055126005, ISSN: 0019-9567, DOI: 10.1128/IAI.01355-08
- H. S. HREGGVIDSDOTTIR ET AL: "The alarmin HMGB1 acts in synergy with endogenous and exogenous danger signals to promote inflammation", JOURNAL OF LEUKOCYTE BIOLOGY, vol. 86, no. 3, 29 June 2009 (2009-06-29), pages 655-662, XP055126006, ISSN: 0741-5400, DOI: 10.1189/jlb.0908548
- MACFARLANE T V ET AL: "PS1-46 Amnion epithelial cells are a source of thymic stromal lymphopoietin", CYTOKINE, ACADEMIC PRESS LTD, PHILADELPHIA, PA, US, vol. 52, no. 1-2, 1 October 2010 (2010-10-01), pages 26-27, XP027261695, ISSN: 1043-4666 [retrieved on 2010-09-02]
- SOUMELIS V ET AL: "Human epithelial cells trigger dendritic cell mediated allergic inflammation by producing TSLP", NATURE IMMUNOLOGY, NATURE PUBLISHING GROUP US, NEW YORK, vol. 3, no. 7, 1 July 2002 (2002-07-01), pages 673-680, XP002364946, ISSN: 1529-2908
- STEVEN F ZIEGLER ET AL: "Sensing the outside world: TSLP regulates barrier immunity", NATURE IMMUNOLOGY, vol. 11, no. 4, 19 March 2010 (2010-03-19) , pages 289-293, XP055453580, New York ISSN: 1529-2908, DOI: 10.1038/ni.1852
- S. I. BOGIATZI ET AL: "Cutting Edge: Proinflammatory and Th2 Cytokines Synergize to Induce Thymic Stromal Lymphopoietin Production by Human Skin Keratinocytes", THE JOURNAL OF IMMUNOLOGY, vol. 178, no. 6, 15 March 2007 (2007-03-15), pages 3373-3377, XP055146979, ISSN: 0022-1767, DOI: 10.4049/jimmunol.178.6.3373
- YANG XIE ET AL: "Longtranscript expression and release of TSLP induced by TLR ligands and cytokines in human keratinocytes", JOURNAL OF DERMATOLOGICAL SCIENCE, ELSEVIER, AMSTERDAM, NL, vol. 66, no. 3, 22 March 2012 (2012-03-22) , pages 233-237, XP028507069, ISSN: 0923-1811, DOI: 10.1016/J.JDERMSCI.2012.03.007 [retrieved on 2012-03-30]

### SEQUENCE LISTING

<110> PIERRE FABRE DERMO-COSMETIQUE
<120> Réactif de stimulation, substitut cutané, explant cutané et procédé
   pour reproduire la physiopathologie de la dermatite atopique
<130> 366011 D32691
<150> FR1357608
   <151> 2013-07-31
<150> FR1361788
   <151> 2013-11-28
<160> 10
<170> PatentIn version 3.5
<210> 1
   <211> 269
   <212> PRT
   <213> homo sapiens
<220>
   <221> MISC_FEATURE
   <223> IL1beta
<400> 1
   Met Ala Glu Val Pro Glu Leu Ala Ser Glu Met Met Ala Tyr Tyr Ser
   1               5                   10                  15
   Gly Asn Glu Asp Asp Leu Phe Phe Glu Ala Asp Gly Pro Lys Gln Met
               20                  25                  30
   Lys Cys Ser Phe Gln Asp Leu Asp Leu Cys Pro Leu Asp Gly Gly Ile
           35                  40                  45
   Gln Leu Arg Ile Ser Asp His His Tyr Ser Lys Gly Phe Arg Gln Ala
       50                  55                  60
   Ala Ser Val Val Val Ala Met Asp Lys Leu Arg Lys Met Leu Val Pro
   65                  70                  75                  80
   Cys Pro Gln Thr Phe Gln Glu Asn Asp Leu Ser Thr Phe Phe Pro Phe
                   85                  90                  95
   Ile Phe Glu Glu Glu Pro Ile Phe Phe Asp Thr Trp Asp Asn Glu Ala
               100                 105                 110
   Tyr Val His Asp Ala Pro Val Arg Ser Leu Asn Cys Thr Leu Arg Asp
           115                 120                 125
   Ser Gln Gln Lys Ser Leu Val Met Ser Gly Pro Tyr Glu Leu Lys Ala
   130 135 140
   Leu His Leu Gln Gly Gln Asp Met Glu Gln Gln Val Val Phe Ser Met
   145 150 155 160
   Ser Phe Val Gln Gly Glu Glu Ser Asn Asp Lys Ile Pro Val Ala Leu
   165 170 175
   Gly Leu Lys Glu Lys Asn Leu Tyr Leu Ser Cys Val Leu Lys Asp Asp
   180 185 190
   Lys Pro Thr Leu Gln Leu Glu Ser Val Asp Pro Lys Asn Tyr Pro Lys
   195 200 205
   Lys Lys Met Glu Lys Arg Phe Val Phe Asn Lys Ile Glu Ile Asn Asn
   210 215 220
   Lys Leu Glu Phe Glu Ser Ala Gln Phe Pro Asn Trp Tyr Ile Ser Thr
   225 230 235 240
   Ser Gln Ala Glu Asn Met Pro Val Phe Leu Gly Gly Thr Lys Gly Gly
   245 250 255
   Gln Asp Ile Thr Asp Phe Thr Met Gln Phe Val Ser Ser
   260 265
<210> 2
   <211> 159
   <212> PRT
   <213> homo sapiens
<220>
   <221> MISC_FEATURE
   <223> TSLP
<400> 2
   Met Phe Pro Phe Ala Leu Leu Tyr Val Leu Ser Val Ser Phe Arg Lys
   1 5 10 15
   Ile Phe Ile Leu Gln Leu Val Gly Leu Val Leu Thr Tyr Asp Phe Thr
   20 25 30
   Asn Cys Asp Phe Glu Lys Ile Lys Ala Ala Tyr Leu Ser Thr Ile Ser
   35 40 45
   Lys Asp Leu Ile Thr Tyr Met Ser Gly Thr Lys Ser Thr Glu Phe Asn
   50 55 60
   Asn Thr Val Ser Cys Ser Asn Arg Pro His Cys Leu Thr Glu Ile Gln
   65                  70                  75                  80
   Ser Leu Thr Phe Asn Pro Thr Ala Gly Cys Ala Ser Leu Ala Lys Glu
                   85                  90                  95
   Met Phe Ala Met Lys Thr Lys Ala Ala Leu Ala Ile Trp Cys Pro Gly
               100                 105                 110
   Tyr Ser Glu Thr Gln Ile Asn Ala Thr Gln Ala Met Lys Lys Arg Arg
           115                 120                 125
   Lys Arg Lys Val Thr Thr Asn Lys Cys Leu Glu Gln Val Ser Gln Leu
       130                 135                 140
   Gln Gly Leu Trp Arg Arg Phe Asn Arg Pro Leu Leu Lys Gln Gln
   145                 150                 155
<210> 3
   <211> 91
   <212> PRT
   <213> homo sapiens
<220>
   <221> MISC_FEATURE
   <223> RANTES
<400> 3
   Met Lys Val Ser Ala Ala Ala Leu Ala Val Ile Leu Ile Ala Thr Ala
   1               5                   10                  15
   Leu Cys Ala Pro Ala Ser Ala Ser Pro Tyr Ser Ser Asp Thr Thr Pro
               20                  25                  30
   Cys Cys Phe Ala Tyr Ile Ala Arg Pro Leu Pro Arg Ala His Ile Lys
           35                  40                  45
   Glu Tyr Phe Tyr Thr Ser Gly Lys Cys Ser Asn Pro Ala Val Val Phe
       50                  55                  60
   Val Thr Arg Lys Asn Arg Gln Val Cys Ala Asn Pro Glu Lys Lys Trp
   65                  70                  75                  80
   Val Arg Glu Tyr Ile Asn Ser Leu Glu Met Ser
                   85                  90
<210> 4
   <211> 99
   <212> PRT
   <213> homo sapiens
<220>
   <221> MISC_FEATURE
   <223> MCP3
<400> 4
   Met Lys Ala Ser Ala Ala Leu Leu Cys Leu Leu Leu Thr Ala Ala Ala
   1               5                   10                  15
   Phe Ser Pro Gln Gly Leu Ala Gln Pro Val Gly Ile Asn Thr Ser Thr
               20                  25                  30
   Thr Cys Cys Tyr Arg Phe Ile Asn Lys Lys Ile Pro Lys Gln Arg Leu
           35                  40                  45
   Glu Ser Tyr Arg Arg Thr Thr Ser Ser His Cys Pro Arg Glu Ala Val
       50                  55                  60
   Ile Phe Lys Thr Lys Leu Asp Lys Glu Ile Cys Ala Asp Pro Thr Gln
   65                  70                  75                  80
   Lys Trp Val Gln Asp Phe Met Lys His Leu Asp Lys Lys Thr Gln Thr
                   85                  90                  95
   Pro Lys Leu
<210> 5
   <211> 212
   <212> PRT
   <213> homo sapiens
<220>
   <221> MISC_FEATURE
   <223> IL-6
<400> 5
   Met Asn Ser Phe Ser Thr Ser Ala Phe Gly Pro Val Ala Phe Ser Leu
   1               5                   10                  15
   Gly Leu Leu Leu Val Leu Pro Ala Ala Phe Pro Ala Pro Val Pro Pro
               20                  25                  30
   Gly Glu Asp Ser Lys Asp Val Ala Ala Pro His Arg Gln Pro Leu Thr
           35                  40                  45
   Ser Ser Glu Arg Ile Asp Lys Gln Ile Arg Tyr Ile Leu Asp Gly Ile
       50                  55                  60
   Ser Ala Leu Arg Lys Glu Thr Cys Asn Lys Ser Asn Met Cys Glu Ser
   65                  70                  75                  80
   Ser Lys Glu Ala Leu Ala Glu Asn Asn Leu Asn Leu Pro Lys Met Ala
                   85                  90                  95
   Glu Lys Asp Gly Cys Phe Gln Ser Gly Phe Asn Glu Glu Thr Cys Leu
               100                 105                 110
   Val Lys Ile Ile Thr Gly Leu Leu Glu Phe Glu Val Tyr Leu Glu Tyr
           115                 120                 125
   Leu Gln Asn Arg Phe Glu Ser Ser Glu Glu Gln Ala Arg Ala Val Gln
       130                 135                 140
   Met Ser Thr Lys Val Leu Ile Gln Phe Leu Gln Lys Lys Ala Lys Asn
   145                 150                 155                 160
   Leu Asp Ala Ile Thr Thr Pro Asp Pro Thr Thr Asn Ala Ser Leu Leu
                   165                 170                 175
   Thr Lys Leu Gln Ala Gln Asn Gln Trp Leu Gln Asp Met Thr Thr His
               180                 185                 190
   Leu Ile Leu Arg Ser Phe Lys Glu Phe Leu Gln Ser Ser Leu Arg Ala
           195                 200                 205
   Leu Arg Gln Met
       210
<210> 6
   <211> 99
   <212> PRT
   <213> homo sapiens
<220>
   <221> MISC_FEATURE
   <223> IL-8
<400> 6
   Met Thr Ser Lys Leu Ala Val Ala Leu Leu Ala Ala Phe Leu Ile Ser
   1               5                   10                  15
   Ala Ala Leu Cys Glu Gly Ala Val Leu Pro Arg Ser Ala Lys Glu Leu
               20                  25                  30
   Arg Cys Gln Cys Ile Lys Thr Tyr Ser Lys Pro Phe His Pro Lys Phe
   35 40 45
   Ile Lys Glu Leu Arg Val Ile Glu Ser Gly Pro His Cys Ala Asn Thr
   50 55 60
   Glu Ile Ile Val Lys Leu Ser Asp Gly Arg Glu Leu Cys Leu Asp Pro
   65 70 75 80
   Lys Glu Asn Trp Val Gln Arg Val Val Glu Lys Phe Leu Lys Arg Ala
   85 90 95
   Glu Asn Ser
<210> 7
   <211> 4061
   <212> PRT
   <213> homo sapiens
<220>
   <221> MISC_FEATURE
   <223> filaggrine
<400> 7
   Met Ser Thr Leu Leu Glu Asn Ile Phe Ala Ile Ile Asn Leu Phe Lys
   1 5 10 15
   Gln Tyr Ser Lys Lys Asp Lys Asn Thr Asp Thr Leu Ser Lys Lys Glu
   20 25 30
   Leu Lys Glu Leu Leu Glu Lys Glu Phe Arg Gln Ile Leu Lys Asn Pro
   35 40 45
   Asp Asp Pro Asp Met Val Asp Val Phe Met Asp His Leu Asp Ile Asp
   50 55 60
   His Asn Lys Lys Ile Asp Phe Thr Glu Phe Leu Leu Met Val Phe Lys
   65 70 75 80
   Leu Ala Gln Ala Tyr Tyr Glu Ser Thr Arg Lys Glu Asn Leu Pro Ile
   85 90 95
   Ser Gly His Lys His Arg Lys His Ser His His Asp Lys His Glu Asp
   100 105 110
   Asn Lys Gln Glu Glu Asn Lys Glu Asn Arg Lys Arg Pro Ser Ser Leu
   115 120 125
   Glu Arg Arg Asn Asn Arg Lys Gly Asn Lys Gly Arg Ser Lys Ser Pro
       130                 135                 140
   Arg Glu Thr Gly Gly Lys Arg His Glu Ser Ser Ser Glu Lys Lys Glu
   145                 150                 155                 160
   Arg Lys Gly Tyr Ser Pro Thr His Arg Glu Glu Glu Tyr Gly Lys Asn
                   165                 170                 175
   His His Asn Ser Ser Lys Lys Glu Lys Asn Lys Thr Glu Asn Thr Arg
               180                 185                 190
   Leu Gly Asp Asn Arg Lys Arg Leu Ser Glu Arg Leu Glu Glu Lys Glu
           195                 200                 205
   Asp Asn Glu Glu Gly Val Tyr Asp Tyr Glu Asn Thr Gly Arg Met Thr
       210                 215                 220
   Gln Lys Trp Ile Gln Ser Gly His Ile Ala Thr Tyr Tyr Thr Ile Gln
   225                 230                 235                 240
   Asp Glu Ala Tyr Asp Thr Thr Asp Ser Leu Leu Glu Glu Asn Lys Ile
                   245                 250                 255
   Tyr Glu Arg Ser Arg Ser Ser Asp Gly Lys Ser Ser Ser Gln Val Asn
               260                 265                 270
   Arg Ser Arg His Glu Asn Thr Ser Gln Val Pro Leu Gln Glu Ser Arg
           275                 280                 285
   Thr Arg Lys Arg Arg Gly Ser Arg Val Ser Gln Asp Arg Asp Ser Glu
       290                 295                 300
   Gly His Ser Glu Asp Ser Glu Arg His Ser Gly Ser Ala Ser Arg Asn
   305                 310                 315                 320
   His His Gly Ser Ala Trp Glu Gln Ser Arg Asp Gly Ser Arg His Pro
                   325                 330                 335
   Arg Ser His Asp Glu Asp Arg Ala Ser His Gly His Ser Ala Asp Ser
               340                 345                 350
   Ser Arg Gln Ser Gly Thr Arg His Ala Glu Thr Ser Ser Arg Gly Gln
           355                 360                 365
   Thr Ala Ser Ser His Glu Gln Ala Arg Ser Ser Pro Gly Glu Arg His
       370                 375                 380
   Gly Ser Gly His Gln Gln Ser Ala Asp Ser Ser Arg His Ser Ala Thr
   385                 390                 395                 400
   Gly Arg Gly Gln Ala Ser Ser Ala Val Ser Asp Arg Gly His Arg Gly
                   405                 410                 415
   Ser Ser Gly Ser Gln Ala Ser Asp Ser Glu Gly His Ser Glu Asn Ser
               420                 425                 430
   Asp Thr Gln Ser Val Ser Gly His Gly Lys Ala Gly Leu Arg Gln Gln
           435                 440                 445
   Ser His Gln Glu Ser Thr Arg Gly Arg Ser Gly Glu Arg Ser Gly Arg
       450                 455                 460
   Ser Gly Ser Ser Leu Tyr Gln Val Ser Thr His Glu Gln Pro Asp Ser
   465                 470                 475                 480
   Ala His Gly Arg Thr Gly Thr Ser Thr Gly Gly Arg Gln Gly Ser His
                   485                 490                 495
   His Glu Gln Ala Arg Asp Ser Ser Arg His Ser Ala Ser Gln Glu Gly
               500                 505                 510
   Gln Asp Thr Ile Arg Gly His Pro Gly Ser Ser Arg Gly Gly Arg Gln
           515                 520                 525
   Gly Ser His His Glu Gln Ser Val Asn Arg Ser Gly His Ser Gly Ser
       530                 535                 540
   His His Ser His Thr Thr Ser Gln Gly Arg Ser Asp Ala Ser His Gly
   545                 550                 555                 560
   Gln Ser Gly Ser Arg Ser Ala Ser Arg Gln Thr Arg Asn Glu Glu Gln
                   565                 570                 575
   Ser Gly Asp Gly Thr Arg His Ser Gly Ser Arg His His Glu Ala Ser
               580                 585                 590
   Ser Gln Ala Asp Ser Ser Arg His Ser Gln Val Gly Gln Gly Gln Ser
           595                 600                 605
   Ser Gly Pro Arg Thr Ser Arg Asn Gln Gly Ser Ser Val Ser Gln Asp
       610                 615                 620
   Ser Asp Ser Gln Gly His Ser Glu Asp Ser Glu Arg Trp Ser Gly Ser
   625                 630                 635                 640
   Ala Ser Arg Asn His His Gly Ser Ala Gln Glu Gln Ser Arg Asp Gly
                   645                 650                 655
   Ser Arg His Pro Arg Ser His His Glu Asp Arg Ala Gly His Gly His
               660                 665                 670
   Ser Ala Asp Ser Ser Arg Lys Ser Gly Thr Arg His Thr Gln Asn Ser
           675                 680                 685
   Ser Ser Gly Gln Ala Ala Ser Ser His Glu Gln Ala Arg Ser Ser Ala
       690                 695                 700
   Gly Glu Arg His Gly Ser Arg His Gln Leu Gln Ser Ala Asp Ser Ser
   705                 710                 715                 720
   Arg His Ser Gly Thr Gly His Gly Gln Ala Ser Ser Ala Val Arg Asp
                   725                 730                 735
   Ser Gly His Arg Gly Ser Ser Gly Ser Gln Ala Thr Asp Ser Glu Gly
               740                 745                 750
   His Ser Glu Asp Ser Asp Thr Gln Ser Val Ser Gly His Gly Gln Ala
           755                 760                 765
   Gly His His Gln Gln Ser His Gln Glu Ser Ala Arg Asp Arg Ser Gly
       770                 775                 780
   Glu Arg Ser Arg Arg Ser Gly Ser Phe Leu Tyr Gln Val Ser Thr His
   785                 790                 795                 800
   Lys Gln Ser Glu Ser Ser His Gly Trp Thr Gly Pro Ser Thr Gly Val
                   805                 810                 815
   Arg Gln Gly Ser His His Glu Gln Ala Arg Asp Asn Ser Arg His Ser
               820                 825                 830
   Ala Ser Gln Asp Gly Gln Asp Thr Ile Arg Gly His Pro Gly Ser Ser
           835                 840                 845
   Arg Arg Gly Arg Gln Gly Ser His His Glu Gln Ser Val Asp Arg Ser
       850                 855                 860
   Gly His Ser Gly Ser His His Ser His Thr Thr Ser Gln Gly Arg Ser
   865                 870                 875                 880
   Asp Ala Ser Arg Gly Gln Ser Gly Ser Arg Ser Ala Ser Arg Thr Thr
                   885                 890                 895
   Arg Asn Glu Glu Gln Ser Arg Asp Gly Ser Arg His Ser Gly Ser Arg
               900                 905                 910
   His His Glu Ala Ser Ser His Ala Asp Ile Ser Arg His Ser Gln Ala
           915                 920                 925
   Gly Gln Gly Gln Ser Glu Gly Ser Arg Thr Ser Arg Arg Gln Gly Ser
       930                 935                 940
   Ser Val Ser Gln Asp Ser Asp Ser Glu Gly His Ser Glu Asp Ser Glu
   945                 950                 955                 960
   Arg Trp Ser Gly Ser Ala Ser Arg Asn His Arg Gly Ser Ala Gln Glu
                   965                 970                 975
   Gln Ser Arg His Gly Ser Arg His Pro Arg Ser His His Glu Asp Arg
               980                 985                 990
   Ala Gly His Gly His Ser Ala Asp  Ser Ser Arg Gln Ser  Gly Thr Pro
           995                 1000                 1005
   His Ala  Glu Thr Ser Ser Gly  Gly Gln Ala Ala Ser  Ser His Glu
       1010                 1015                 1020
   Gln Ala  Arg Ser Ser Pro Gly  Glu Arg His Gly Ser  Arg His Gln
       1025                 1030                 1035
   Gln Ser  Ala Asp Ser Ser Arg  His Ser Gly Ile Pro  Arg Arg Gln
       1040                 1045                 1050
   Ala Ser  Ser Ala Val Arg Asp  Ser Gly His Trp Gly  Ser Ser Gly
       1055                 1060                 1065
   Ser Gln  Ala Ser Asp Ser Glu  Gly His Ser Glu Glu  Ser Asp Thr
       1070                 1075                 1080
   Gln Ser  Val Ser Gly His Gly  Gln Asp Gly Pro His  Gln Gln Ser
       1085                 1090                 1095
   His Gln  Glu Ser Ala Arg Asp  Trp Ser Gly Gly Arg  Ser Gly Arg
       1100                 1105                 1110
   Ser Gly  Ser Phe Ile Tyr Gln  Val Ser Thr His Glu  Gln Ser Glu
       1115                 1120                 1125
   Ser Ala  His Gly Arg Thr Arg  Thr Ser Thr Gly Arg  Arg Gln Gly
       1130                 1135                 1140
   Ser His  His Glu Gln Ala Arg  Asp Ser Ser Arg His  Ser Ala Ser
       1145                 1150                 1155
   Gln Glu  Gly Gln Asp Thr Ile  Arg Ala His Pro Gly  Ser Arg Arg
       1160                 1165                 1170
   Gly Gly  Arg Gln Gly Ser His  His Glu Gln Ser Val  Asp Arg Ser
       1175                 1180                 1185
   Gly His  Ser Gly Ser His His  Ser His Thr Thr Ser  Gln Gly Arg
       1190                 1195                 1200
   Ser Asp  Ala Ser His Gly Gln  Ser Gly Ser Arg Ser  Ala Ser Arg
       1205                 1210                 1215
   Gln Thr  Arg Lys Asp Lys Gln  Ser Gly Asp Gly Ser  Arg His Ser
       1220                 1225                 1230
   Gly Ser  Arg His His Glu Ala  Ala Ser Trp Ala Asp  Ser Ser Arg
       1235                 1240                 1245
   His Ser  Gln Val Gly Gln Glu  Gln Ser Ser Gly Ser  Arg Thr Ser
       1250                 1255                 1260
   Arg His  Gln Gly Ser Ser Val  Ser Gln Asp Ser Asp  Ser Glu Arg
       1265                 1270                 1275
   His Ser  Asp Asp Ser Glu Arg  Leu Ser Gly Ser Ala  Ser Arg Asn
       1280                 1285                 1290
   His His  Gly Ser Ser Arg Glu  Gln Ser Arg Asp Gly  Ser Arg His
       1295                 1300                 1305
   Pro Gly  Phe His Gln Glu Asp  Arg Ala Ser His Gly  His Ser Ala
       1310                 1315                 1320
   Asp Ser  Ser Arg Gln Ser Gly  Thr His His Thr Glu  Ser Ser Ser
       1325                 1330                 1335
   His Gly  Gln Ala Val Ser Ser  His Glu Gln Ala Arg  Ser Ser Pro
       1340                 1345                 1350
   Gly Glu  Arg His Gly Ser Arg  His Gln Gln Ser Ala  Asp Ser Ser
       1355                 1360                 1365
   Arg His Ser Gly Ile Gly His Arg Gln Ala Ser Ser Ala Val Arg
       1370                1375                1380
   Asp Ser Gly His Arg Gly Ser Ser Gly Ser Gln Val Thr Asn Ser
       1385                1390                1395
   Glu Gly His Ser Glu Asp Ser Asp Thr Gln Ser Val Ser Ala His
       1400                1405                1410
   Gly Gln Ala Gly Pro His Gln Gln Ser His Lys Glu Ser Ala Arg
       1415                1420                1425
   Gly Gln Ser Gly Glu Ser Ser Gly Arg Ser Arg Ser Phe Leu Tyr
       1430                1435                1440
   Gln Val Ser Ser His Glu Gln Ser Glu Ser Thr His Gly Gln Thr
       1445                1450                1455
   Ala Pro Ser Thr Gly Gly Arg Gln Gly Ser Arg His Glu Gln Ala
       1460                1465                1470
   Arg Asn Ser Ser Arg His Ser Ala Ser Gln Asp Gly Gln Asp Thr
       1475                1480                1485
   Ile Arg Gly His Pro Gly Ser Ser Arg Gly Gly Arg Gln Gly Ser
       1490                1495                1500
   Tyr His Glu Gln Ser Val Asp Arg Ser Gly His Ser Gly Tyr His
       1505                1510                1515
   His Ser His Thr Thr Pro Gln Gly Arg Ser Asp Ala Ser His Gly
       1520                1525                1530
   Gln Ser Gly Pro Arg Ser Ala Ser Arg Gln Thr Arg Asn Glu Glu
       1535                1540                1545
   Gln Ser Gly Asp Gly Ser Arg His Ser Gly Ser Arg His His Glu
       1550                1555                1560
   Pro Ser Thr Arg Ala Gly Ser Ser Arg His Ser Gln Val Gly Gln
       1565                1570                1575
   Gly Glu Ser Ala Gly Ser Lys Thr Ser Arg Arg Gln Gly Ser Ser
       1580                1585                1590
   Val Ser Gln Asp Arg Asp Ser Glu Gly His Ser Glu Asp Ser Glu
   1595 1600 1605
   Arg Arg Ser Glu Ser Ala Ser Arg Asn His Tyr Gly Ser Ala Arg
   1610 1615 1620
   Glu Gln Ser Arg His Gly Ser Arg Asn Pro Arg Ser His Gln Glu
   1625 1630 1635
   Asp Arg Ala Ser His Gly His Ser Ala Glu Ser Ser Arg Gln Ser
   1640 1645 1650
   Gly Thr Arg His Ala Glu Thr Ser Ser Gly Gly Gln Ala Ala Ser
   1655 1660 1665
   Ser Gln Glu Gln Ala Arg Ser Ser Pro Gly Glu Arg His Gly Ser
   1670 1675 1680
   Arg His Gln Gln Ser Ala Asp Ser Ser Thr Asp Ser Gly Thr Gly
   1685 1690 1695
   Arg Arg Gln Asp Ser Ser Val Val Gly Asp Ser Gly Asn Arg Gly
   1700 1705 1710
   Ser Ser Gly Ser Gln Ala Ser Asp Ser Glu Gly His Ser Glu Glu
   1715 1720 1725
   Ser Asp Thr Gln Ser Val Ser Ala His Gly Gln Ala Gly Pro His
   1730 1735 1740
   Gln Gln Ser His Gln Glu Ser Thr Arg Gly Gln Ser Gly Glu Arg
   1745 1750 1755
   Ser Gly Arg Ser Gly Ser Phe Leu Tyr Gln Val Ser Thr His Glu
   1760 1765 1770
   Gln Ser Glu Ser Ala His Gly Arg Thr Gly Pro Ser Thr Gly Gly
   1775 1780 1785
   Arg Gln Arg Ser Arg His Glu Gln Ala Arg Asp Ser Ser Arg His
   1790 1795 1800
   Ser Ala Ser Gln Glu Gly Gln Asp Thr Ile Arg Gly His Pro Gly
   1805 1810 1815
   Ser Ser Arg Gly Gly Arg Gln Gly Ser His Tyr Glu Gln Ser Val
   1820 1825 1830
   Asp Ser  Ser Gly His Ser Gly  Ser His His Ser His  Thr Thr Ser
       1835                 1840                 1845
   Gln Glu  Arg Ser Asp Val Ser  Arg Gly Gln Ser Gly  Ser Arg Ser
       1850                 1855                 1860
   Val Ser  Arg Gln Thr Arg Asn  Glu Lys Gln Ser Gly  Asp Gly Ser
       1865                 1870                 1875
   Arg His  Ser Gly Ser Arg His  His Glu Ala Ser Ser  Arg Ala Asp
       1880                 1885                 1890
   Ser Ser  Arg His Ser Gln Val  Gly Gln Gly Gln Ser  Ser Gly Pro
       1895                 1900                 1905
   Arg Thr  Ser Arg Asn Gln Gly  Ser Ser Val Ser Gln  Asp Ser Asp
       1910                 1915                 1920
   Ser Gln  Gly His Ser Glu Asp  Ser Glu Arg Trp Ser  Gly Ser Ala
       1925                 1930                 1935
   Ser Arg  Asn His Leu Gly Ser  Ala Trp Glu Gln Ser  Arg Asp Gly
       1940                 1945                 1950
   Ser Arg  His Pro Gly Ser His  His Glu Asp Arg Ala  Gly His Gly
       1955                 1960                 1965
   His Ser  Ala Asp Ser Ser Arg  Gln Ser Gly Thr Arg  His Thr Glu
       1970                 1975                 1980
   Ser Ser  Ser Arg Gly Gln Ala  Ala Ser Ser His Glu  Gln Ala Arg
       1985                 1990                 1995
   Ser Ser  Ala Gly Glu Arg His  Gly Ser His His Gln  Leu Gln Ser
       2000                 2005                 2010
   Ala Asp  Ser Ser Arg His Ser  Gly Ile Gly His Gly  Gln Ala Ser
       2015                 2020                 2025
   Ser Ala  Val Arg Asp Ser Gly  His Arg Gly Tyr Ser  Gly Ser Gln
       2030                 2035                 2040
   Ala Ser  Asp Ser Glu Gly His  Ser Glu Asp Ser Asp  Thr Gln Ser
       2045                 2050                 2055
   Val Ser  Ala Gln Gly Lys Ala  Gly Pro His Gln Gln  Ser His Lys
       2060                 2065                 2070
   Glu Ser Ala Arg Gly Gln Ser Gly Glu Ser Ser Gly Arg Ser Gly
   2075 2080 2085
   Ser Phe Leu Tyr Gln Val Ser Thr His Glu Gln Ser Glu Ser Thr
   2090 2095 2100
   His Gly Gln Ser Ala Pro Ser Thr Gly Gly Arg Gln Gly Ser His
   2105 2110 2115
   Tyr Asp Gln Ala Gln Asp Ser Ser Arg His Ser Ala Ser Gln Glu
   2120 2125 2130
   Gly Gln Asp Thr Ile Arg Gly His Pro Gly Pro Ser Arg Gly Gly
   2135 2140 2145
   Arg Gln Gly Ser His Gln Glu Gln Ser Val Asp Arg Ser Gly His
   2150 2155 2160
   Ser Gly Ser His His Ser His Thr Thr Ser Gln Gly Arg Ser Asp
   2165 2170 2175
   Ala Ser Arg Gly Gln Ser Gly Ser Arg Ser Ala Ser Arg Lys Thr
   2180 2185 2190
   Tyr Asp Lys Glu Gln Ser Gly Asp Gly Ser Arg His Ser Gly Ser
   2195 2200 2205
   His His His Glu Ala Ser Ser Trp Ala Asp Ser Ser Arg His Ser
   2210 2215 2220
   Leu Val Gly Gln Gly Gln Ser Ser Gly Pro Arg Thr Ser Arg Pro
   2225 2230 2235
   Arg Gly Ser Ser Val Ser Gln Asp Ser Asp Ser Glu Gly His Ser
   2240 2245 2250
   Glu Asp Ser Glu Arg Arg Ser Gly Ser Ala Ser Arg Asn His His
   2255 2260 2265
   Gly Ser Ala Gln Glu Gln Ser Arg Asp Gly Ser Arg His Pro Arg
   2270 2275 2280
   Ser His His Glu Asp Arg Ala Gly His Gly His Ser Ala Glu Ser
   2285 2290 2295
   Ser Arg Gln Ser Gly Thr His His Ala Glu Asn Ser Ser Gly Gly
   2300 2305 2310
   Gln Ala Ala Ser Ser His Glu Gln Ala Arg Ser Ser Ala Gly Glu
   2315 2320 2325
   Arg His Gly Ser His His Gln Gln Ser Ala Asp Ser Ser Arg His
   2330 2335 2340
   Ser Gly Ile Gly His Gly Gln Ala Ser Ser Ala Val Arg Asp Ser
   2345 2350 2355
   Gly His Arg Gly Ser Ser Gly Ser Gln Ala Ser Asp Ser Glu Gly
   2360 2365 2370
   His Ser Glu Asp Ser Asp Thr Gln Ser Val Ser Ala His Gly Gln
   2375 2380 2385
   Ala Gly Pro His Gln Gln Ser His Gln Glu Ser Thr Arg Gly Arg
   2390 2395 2400
   Ser Ala Gly Arg Ser Gly Arg Ser Gly Ser Phe Leu Tyr Gln Val
   2405 2410 2415
   Ser Thr His Glu Gln Ser Glu Ser Ala His Gly Arg Thr Gly Thr
   2420 2425 2430
   Ser Thr Gly Gly Arg Gln Gly Ser His His Lys Gln Ala Arg Asp
   2435 2440 2445
   Ser Ser Arg His Ser Thr Ser Gln Glu Gly Gln Asp Thr Ile His
   2450 2455 2460
   Gly His Pro Gly Ser Ser Ser Gly Gly Arg Gln Gly Ser His Tyr
   2465 2470 2475
   Glu Gln Leu Val Asp Arg Ser Gly His Ser Gly Ser His His Ser
   2480 2485 2490
   His Thr Thr Ser Gln Gly Arg Ser Asp Ala Ser His Gly His Ser
   2495 2500 2505
   Gly Ser Arg Ser Ala Ser Arg Gln Thr Arg Asn Asp Glu Gln Ser
   2510 2515 2520
   Gly Asp Gly Ser Arg His Ser Gly Ser Arg His His Glu Ala Ser
   2525 2530 2535
   Ser Arg Ala Asp Ser Ser Gly His Ser Gln Val Gly Gln Gly Gln
   2540 2545 2550
   Ser Glu Gly Pro Arg Thr Ser Arg Asn Trp Gly Ser Ser Phe Ser
   2555 2560 2565
   Gln Asp Ser Asp Ser Gln Gly His Ser Glu Asp Ser Glu Arg Trp
   2570 2575 2580
   Ser Gly Ser Ala Ser Arg Asn His His Gly Ser Ala Gln Glu Gln
   2585 2590 2595
   Leu Arg Asp Gly Ser Arg His Pro Arg Ser His Gln Glu Asp Arg
   2600 2605 2610
   Ala Gly His Gly His Ser Ala Asp Ser Ser Arg Gln Ser Gly Thr
   2615 2620 2625
   Arg His Thr Gln Thr Ser Ser Gly Gly Gln Ala Ala Ser Ser His
   2630 2635 2640
   Glu Gln Ala Arg Ser Ser Ala Gly Glu Arg His Gly Ser His His
   2645 2650 2655
   Gln Gln Ser Ala Asp Ser Ser Arg His Ser Gly Ile Gly His Gly
   2660 2665 2670
   Gln Ala Ser Ser Ala Val Arg Asp Ser Gly His Arg Gly Tyr Ser
   2675 2680 2685
   Gly Ser Gln Ala Ser Asp Asn Glu Gly His Ser Glu Asp Ser Asp
   2690 2695 2700
   Thr Gln Ser Val Ser Ala His Gly Gln Ala Gly Ser His Gln Gln
   2705 2710 2715
   Ser His Gln Glu Ser Ala Arg Gly Arg Ser Gly Glu Thr Ser Gly
   2720 2725 2730
   His Ser Gly Ser Phe Leu Tyr Gln Val Ser Thr His Glu Gln Ser
   2735 2740 2745
   Glu Ser Ser His Gly Trp Thr Gly Pro Ser Thr Arg Gly Arg Gln
   2750 2755 2760
   Gly Ser Arg His Glu Gln Ala Gln Asp Ser Ser Arg His Ser Ala
   2765 2770 2775
   Ser Gln Asp Gly Gln Asp Thr Ile Arg Gly His Pro Gly Ser Ser
       2780                2785                2790
   Arg Gly Gly Arg Gln Gly Tyr His His Glu His Ser Val Asp Ser
       2795                2800                2805
   Ser Gly His Ser Gly Ser His His Ser His Thr Thr Ser Gln Gly
       2810                2815                2820
   Arg Ser Asp Ala Ser Arg Gly Gln Ser Gly Ser Arg Ser Ala Ser
       2825                2830                2835
   Arg Thr Thr Arg Asn Glu Glu Gln Ser Gly Asp Gly Ser Arg His
       2840                2845                2850
   Ser Gly Ser Arg His His Glu Ala Ser Thr His Ala Asp Ile Ser
       2855                2860                2865
   Arg His Ser Gln Ala Val Gln Gly Gln Ser Glu Gly Ser Arg Arg
       2870                2875                2880
   Ser Arg Arg Gln Gly Ser Ser Val Ser Gln Asp Ser Asp Ser Glu
       2885                2890                2895
   Gly His Ser Glu Asp Ser Glu Arg Trp Ser Gly Ser Ala Ser Arg
       2900                2905                2910
   Asn His His Gly Ser Ala Gln Glu Gln Leu Arg Asp Gly Ser Arg
       2915                2920                2925
   His Pro Arg Ser His Gln Glu Asp Arg Ala Gly His Gly His Ser
       2930                2935                2940
   Ala Asp Ser Ser Arg Gln Ser Gly Thr Arg His Thr Gln Thr Ser
       2945                2950                2955
   Ser Gly Gly Gln Ala Ala Ser Ser His Glu Gln Ala Arg Ser Ser
       2960                2965                2970
   Ala Gly Glu Arg His Gly Ser His His Gln Gln Ser Ala Asp Ser
       2975                2980                2985
   Ser Arg His Ser Gly Ile Gly His Gly Gln Ala Ser Ser Ala Val
       2990                2995                3000
   Arg Asp Ser Gly His Arg Gly Tyr Ser Gly Ser Gln Ala Ser Asp
       3005                3010                3015
   Asn Glu Gly His Ser Glu Asp Ser Asp Thr Gln Ser Val Ser Ala
   3020 3025 3030
   His Gly Gln Ala Gly Ser His Gln Gln Ser His Gln Glu Ser Ala
   3035 3040 3045
   Arg Gly Arg Ser Gly Glu Thr Ser Gly His Ser Gly Ser Phe Leu
   3050 3055 3060
   Tyr Gln Val Ser Thr His Glu Gln Ser Glu Ser Ser His Gly Trp
   3065 3070 3075
   Thr Gly Pro Ser Thr Arg Gly Arg Gln Gly Ser Arg His Glu Gln
   3080 3085 3090
   Ala Gln Asp Ser Ser Arg His Ser Ala Ser Gln Tyr Gly Gln Asp
   3095 3100 3105
   Thr Ile Arg Gly His Pro Gly Ser Ser Arg Gly Gly Arg Gln Gly
   3110 3115 3120
   Tyr His His Glu His Ser Val Asp Ser Ser Gly His Ser Gly Ser
   3125 3130 3135
   His His Ser His Thr Thr Ser Gln Gly Arg Ser Asp Ala Ser Arg
   3140 3145 3150
   Gly Gln Ser Gly Ser Arg Ser Ala Ser Arg Thr Thr Arg Asn Glu
   3155 3160 3165
   Glu Gln Ser Gly Asp Ser Ser Arg His Ser Val Ser Arg His His
   3170 3175 3180
   Glu Ala Ser Thr His Ala Asp Ile Ser Arg His Ser Gln Ala Val
   3185 3190 3195
   Gln Gly Gln Ser Glu Gly Ser Arg Arg Ser Arg Arg Gln Gly Ser
   3200 3205 3210
   Ser Val Ser Gln Asp Ser Asp Ser Glu Gly His Ser Glu Asp Ser
   3215 3220 3225
   Glu Arg Trp Ser Gly Ser Ala Ser Arg Asn His Arg Gly Ser Val
   3230 3235 3240
   Gln Glu Gln Ser Arg His Gly Ser Arg His Pro Arg Ser His His
   3245 3250 3255
   Glu Asp Arg Ala Gly His Gly His Ser Ala Asp Arg Ser Arg Gln
   3260 3265 3270
   Ser Gly Thr Arg His Ala Glu Thr Ser Ser Gly Gly Gln Ala Ala
   3275 3280 3285
   Ser Ser His Glu Gln Ala Arg Ser Ser Pro Gly Glu Arg His Gly
   3290 3295 3300
   Ser Arg His Gln Gln Ser Ala Asp Ser Ser Arg His Ser Gly Ile
   3305 3310 3315
   Pro Arg Gly Gln Ala Ser Ser Ala Val Arg Asp Ser Arg His Trp
   3320 3325 3330
   Gly Ser Ser Gly Ser Gln Ala Ser Asp Ser Glu Gly His Ser Glu
   3335 3340 3345
   Glu Ser Asp Thr Gln Ser Val Ser Gly His Gly Gln Ala Gly Pro
   3350 3355 3360
   His Gln Gln Ser His Gln Glu Ser Ala Arg Asp Arg Ser Gly Gly
   3365 3370 3375
   Arg Ser Gly Arg Ser Gly Ser Phe Leu Tyr Gln Val Ser Thr His
   3380 3385 3390
   Glu Gln Ser Glu Ser Ala His Gly Arg Thr Arg Thr Ser Thr Gly
   3395 3400 3405
   Arg Arg Gln Gly Ser His His Glu Gln Ala Arg Asp Ser Ser Arg
   3410 3415 3420
   His Ser Ala Ser Gln Glu Gly Gln Asp Thr Ile Arg Gly His Pro
   3425 3430 3435
   Gly Ser Ser Arg Arg Gly Arg Gln Gly Ser His Tyr Glu Gln Ser
   3440 3445 3450
   Val Asp Arg Ser Gly His Ser Gly Ser His His Ser His Thr Thr
   3455 3460 3465
   Ser Gln Gly Arg Ser Asp Ala Ser Arg Gly Gln Ser Gly Ser Arg
   3470 3475 3480
   Ser Ala Ser Arg Gln Thr Arg Asn Asp Glu Gln Ser Gly Asp Gly
   3485 3490 3495
   Ser Arg His Ser Trp Ser His His His Glu Ala Ser Thr Gln Ala
   3500 3505 3510
   Asp Ser Ser Arg His Ser Gln Ser Gly Gln Gly Gln Ser Ala Gly
   3515 3520 3525
   Pro Arg Thr Ser Arg Asn Gln Gly Ser Ser Val Ser Gln Asp Ser
   3530 3535 3540
   Asp Ser Gln Gly His Ser Glu Asp Ser Glu Arg Trp Ser Gly Ser
   3545 3550 3555
   Ala Ser Arg Asn His Arg Gly Ser Ala Gln Glu Gln Ser Arg Asp
   3560 3565 3570
   Gly Ser Arg His Pro Thr Ser His His Glu Asp Arg Ala Gly His
   3575 3580 3585
   Gly His Ser Ala Glu Ser Ser Arg Gln Ser Gly Thr His His Ala
   3590 3595 3600
   Glu Asn Ser Ser Gly Gly Gln Ala Ala Ser Ser His Glu Gln Ala
   3605 3610 3615
   Arg Ser Ser Ala Gly Glu Arg His Gly Ser His His Gln Gln Ser
   3620 3625 3630
   Ala Asp Ser Ser Arg His Ser Gly Ile Gly His Gly Gln Ala Ser
   3635 3640 3645
   Ser Ala Val Arg Asp Ser Gly His Arg Gly Ser Ser Gly Ser Gln
   3650 3655 3660
   Ala Ser Asp Ser Glu Gly His Ser Glu Asp Ser Asp Thr Gln Ser
   3665 3670 3675
   Val Ser Ala His Gly Gln Ala Gly Pro His Gln Gln Ser His Gln
   3680 3685 3690
   Glu Ser Thr Arg Gly Arg Ser Ala Gly Arg Ser Gly Arg Ser Gly
   3695 3700 3705
   Ser Phe Leu Tyr Gln Val Ser Thr His Glu Gln Ser Glu Ser Ala
   3710 3715 3720
   His Gly Arg Ala Gly Pro Ser Thr Gly Gly Arg Gln Gly Ser Arg
   3725 3730 3735
   His Glu Gln Ala Arg Asp Ser Ser Arg His Ser Ala Ser Gln Glu
   3740 3745 3750
   Gly Gln Asp Thr Ile Arg Gly His Pro Gly Ser Arg Arg Gly Gly
   3755 3760 3765
   Arg Gln Gly Ser Tyr His Glu Gln Ser Val Asp Arg Ser Gly His
   3770 3775 3780
   Ser Gly Ser His His Ser His Thr Thr Ser Gln Gly Arg Ser Asp
   3785 3790 3795
   Ala Ser His Gly Gln Ser Gly Ser Arg Ser Ala Ser Arg Glu Thr
   3800 3805 3810
   Arg Asn Glu Glu Gln Ser Gly Asp Gly Ser Arg His Ser Gly Ser
   3815 3820 3825
   Arg His His Glu Ala Ser Thr Gln Ala Asp Ser Ser Arg His Ser
   3830 3835 3840
   Gln Ser Gly Gln Gly Glu Ser Ala Gly Ser Arg Arg Ser Arg Arg
   3845 3850 3855
   Gln Gly Ser Ser Val Ser Gln Asp Ser Asp Ser Glu Ala Tyr Pro
   3860 3865 3870
   Glu Asp Ser Glu Arg Arg Ser Glu Ser Ala Ser Arg Asn His His
   3875 3880 3885
   Gly Ser Ser Arg Glu Gln Ser Arg Asp Gly Ser Arg His Pro Gly
   3890 3895 3900
   Ser Ser His Arg Asp Thr Ala Ser His Val Gln Ser Ser Pro Val
   3905 3910 3915
   Gln Ser Asp Ser Ser Thr Ala Lys Glu His Gly His Phe Ser Ser
   3920 3925 3930
   Leu Ser Gln Asp Ser Ala Tyr His Ser Gly Ile Gln Ser Arg Gly
   3935 3940 3945
   Ser Pro His Ser Ser Ser Ser Tyr His Tyr Gln Ser Glu Gly Thr
   3950 3955 3960
   Glu Arg  Gln Lys Gly Gln Ser  Gly Leu Val Trp Arg  His Gly Ser
       3965                 3970                 3975
   Tyr Gly  Ser Ala Asp Tyr Asp  Tyr Gly Glu Ser Gly  Phe Arg His
       3980                 3985                 3990
   Ser Gln  His Gly Ser Val Ser  Tyr Asn Ser Asn Pro  Val Val Phe
       3995                 4000                 4005
   Lys Glu  Arg Ser Asp Ile Cys  Lys Ala Ser Ala Phe  Gly Lys Asp
       4010                 4015                 4020
   His Pro  Arg Tyr Tyr Ala Thr  Tyr Ile Asn Lys Asp  Pro Gly Leu
       4025                 4030                 4035
   Cys Gly  His Ser Ser Asp Ile  Ser Lys Gln Leu Gly  Phe Ser Gln
       4040                 4045                 4050
   Ser Gln  Arg Tyr Tyr Tyr Tyr  Glu
       4055                 4060
<210> 8
   <211> 211
   <212> PRT
   <213> homo sapiens
<220>
   <221> MISC_FEATURE
   <223> claudine 1
<400> 8
   Met Ala Asn Ala Gly Leu Gln Leu Leu Gly Phe Ile Leu Ala Phe Leu
   1               5                   10                  15
   Gly Trp Ile Gly Ala Ile Val Ser Thr Ala Leu Pro Gln Trp Arg Ile
               20                  25                  30
   Tyr Ser Tyr Ala Gly Asp Asn Ile Val Thr Ala Gln Ala Met Tyr Glu
           35                  40                  45
   Gly Leu Trp Met Ser Cys Val Ser Gln Ser Thr Gly Gln Ile Gln Cys
       50                  55                  60
   Lys Val Phe Asp Ser Leu Leu Asn Leu Ser Ser Thr Leu Gln Ala Thr
   65                  70                  75                  80
   Arg Ala Leu Met Val Val Gly Ile Leu Leu Gly Val Ile Ala Ile Phe
                   85                  90                  95
   Val Ala Thr Val Gly Met Lys Cys Met Lys Cys Leu Glu Asp Asp Glu
   100 105 110
   Val Gln Lys Met Arg Met Ala Val Ile Gly Gly Ala Ile Phe Leu Leu
   115 120 125
   Ala Gly Leu Ala Ile Leu Val Ala Thr Ala Trp Tyr Gly Asn Arg Ile
   130 135 140
   Val Gln Glu Phe Tyr Asp Pro Met Thr Pro Val Asn Ala Arg Tyr Glu
   145 150 155 160
   Phe Gly Gln Ala Leu Phe Thr Gly Trp Ala Ala Ala Ser Leu Cys Leu
   165 170 175
   Leu Gly Gly Ala Leu Leu Cys Cys Ser Cys Pro Arg Lys Thr Thr Ser
   180 185 190
   Tyr Pro Thr Pro Arg Pro Tyr Pro Lys Pro Ala Pro Ser Ser Gly Lys
   195 200 205
   Asp Tyr Val
   210
<210> 9
   <211> 784
   <212> PRT
   <213> homo sapiens
<220>
   <221> MISC_FEATURE
   <223> TLR2
<400> 9
   Met Pro His Thr Leu Trp Met Val Trp Val Leu Gly Val Ile Ile Ser
   1 5 10 15
   Leu Ser Lys Glu Glu Ser Ser Asn Gln Ala Ser Leu Ser Cys Asp Arg
   20 25 30
   Asn Gly Ile Cys Lys Gly Ser Ser Gly Ser Leu Asn Ser Ile Pro Ser
   35 40 45
   Gly Leu Thr Glu Ala Val Lys Ser Leu Asp Leu Ser Asn Asn Arg Ile
   50 55 60
   Thr Tyr Ile Ser Asn Ser Asp Leu Gln Arg Cys Val Asn Leu Gln Ala
   65                  70                  75                  80
   Leu Val Leu Thr Ser Asn Gly Ile Asn Thr Ile Glu Glu Asp Ser Phe
                   85                  90                  95
   Ser Ser Leu Gly Ser Leu Glu His Leu Asp Leu Ser Tyr Asn Tyr Leu
               100                 105                 110
   Ser Asn Leu Ser Ser Ser Trp Phe Lys Pro Leu Ser Ser Leu Thr Phe
           115                 120                 125
   Leu Asn Leu Leu Gly Asn Pro Tyr Lys Thr Leu Gly Glu Thr Ser Leu
       130                 135                 140
   Phe Ser His Leu Thr Lys Leu Gln Ile Leu Arg Val Gly Asn Met Asp
   145                 150                 155                 160
   Thr Phe Thr Lys Ile Gln Arg Lys Asp Phe Ala Gly Leu Thr Phe Leu
                   165                 170                 175
   Glu Glu Leu Glu Ile Asp Ala Ser Asp Leu Gln Ser Tyr Glu Pro Lys
               180                 185                 190
   Ser Leu Lys Ser Ile Gln Asn Val Ser His Leu Ile Leu His Met Lys
           195                 200                 205
   Gln His Ile Leu Leu Leu Glu Ile Phe Val Asp Val Thr Ser Ser Val
       210                 215                 220
   Glu Cys Leu Glu Leu Arg Asp Thr Asp Leu Asp Thr Phe His Phe Ser
   225                 230                 235                 240
   Glu Leu Ser Thr Gly Glu Thr Asn Ser Leu Ile Lys Lys Phe Thr Phe
                   245                 250                 255
   Arg Asn Val Lys Ile Thr Asp Glu Ser Leu Phe Gln Val Met Lys Leu
               260                 265                 270
   Leu Asn Gln Ile Ser Gly Leu Leu Glu Leu Glu Phe Asp Asp Cys Thr
           275                 280                 285
   Leu Asn Gly Val Gly Asn Phe Arg Ala Ser Asp Asn Asp Arg Val Ile
       290                 295                 300
   Asp Pro Gly Lys Val Glu Thr Leu Thr Ile Arg Arg Leu His Ile Pro
   305                 310                 315                 320
   Arg Phe Tyr Leu Phe Tyr Asp Leu Ser Thr Leu Tyr Ser Leu Thr Glu
                   325                 330                 335
   Arg Val Lys Arg Ile Thr Val Glu Asn Ser Lys Val Phe Leu Val Pro
               340                 345                 350
   Cys Leu Leu Ser Gln His Leu Lys Ser Leu Glu Tyr Leu Asp Leu Ser
           355                 360                 365
   Glu Asn Leu Met Val Glu Glu Tyr Leu Lys Asn Ser Ala Cys Glu Asp
       370                 375                 380
   Ala Trp Pro Ser Leu Gln Thr Leu Ile Leu Arg Gln Asn His Leu Ala
   385                 390                 395                 400
   Ser Leu Glu Lys Thr Gly Glu Thr Leu Leu Thr Leu Lys Asn Leu Thr
                   405                 410                 415
   Asn Ile Asp Ile Ser Lys Asn Ser Phe His Ser Met Pro Glu Thr Cys
               420                 425                 430
   Gln Trp Pro Glu Lys Met Lys Tyr Leu Asn Leu Ser Ser Thr Arg Ile
           435                 440                 445
   His Ser Val Thr Gly Cys Ile Pro Lys Thr Leu Glu Ile Leu Asp Val
       450                 455                 460
   Ser Asn Asn Asn Leu Asn Leu Phe Ser Leu Asn Leu Pro Gln Leu Lys
   465                 470                 475                 480
   Glu Leu Tyr Ile Ser Arg Asn Lys Leu Met Thr Leu Pro Asp Ala Ser
                   485                 490                 495
   Leu Leu Pro Met Leu Leu Val Leu Lys Ile Ser Arg Asn Ala Ile Thr
               500                 505                 510
   Thr Phe Ser Lys Glu Gln Leu Asp Ser Phe His Thr Leu Lys Thr Leu
           515                 520                 525
   Glu Ala Gly Gly Asn Asn Phe Ile Cys Ser Cys Glu Phe Leu Ser Phe
       530                 535                 540
   Thr Gln Glu Gln Gln Ala Leu Ala Lys Val Leu Ile Asp Trp Pro Ala
   545                 550                 555                 560
   Asn Tyr Leu Cys Asp Ser Pro Ser His Val Arg Gly Gln Gln Val Gln
                   565                 570                 575
   Asp Val Arg Leu Ser Val Ser Glu Cys His Arg Thr Ala Leu Val Ser
   580 585 590
   Gly Met Cys Cys Ala Leu Phe Leu Leu Ile Leu Leu Thr Gly Val Leu
   595 600 605
   Cys His Arg Phe His Gly Leu Trp Tyr Met Lys Met Met Trp Ala Trp
   610 615 620
   Leu Gln Ala Lys Arg Lys Pro Arg Lys Ala Pro Ser Arg Asn Ile Cys
   625 630 635 640
   Tyr Asp Ala Phe Val Ser Tyr Ser Glu Arg Asp Ala Tyr Trp Val Glu
   645 650 655
   Asn Leu Met Val Gln Glu Leu Glu Asn Phe Asn Pro Pro Phe Lys Leu
   660 665 670
   Cys Leu His Lys Arg Asp Phe Ile Pro Gly Lys Trp Ile Ile Asp Asn
   675 680 685
   Ile Ile Asp Ser Ile Glu Lys Ser His Lys Thr Val Phe Val Leu Ser
   690 695 700
   Glu Asn Phe Val Lys Ser Glu Trp Cys Lys Tyr Glu Leu Asp Phe Ser
   705 710 715 720
   His Phe Arg Leu Phe Asp Glu Asn Asn Asp Ala Ala Ile Leu Ile Leu
   725 730 735
   Leu Glu Pro Ile Glu Lys Lys Ala Ile Pro Gln Arg Phe Cys Lys Leu
   740 745 750
   Arg Lys Ile Met Asn Thr Lys Thr Tyr Leu Glu Trp Pro Met Asp Glu
   755 760 765
   Ala Gln Arg Glu Gly Phe Trp Val Asn Leu Arg Ala Ala Ile Lys Ser
   770 775 780
<210> 10
   <211> 904
   <212> PRT
   <213> homo sapiens
<220>
   <221> MISC_FEATURE
   <223> TLR3
<400> 10

## Description

### Résumé de l'invention

La présente demande vise un nouveau modèle tissulaire *in vitro* qui reproduit des caractéristiques moléculaires et structurales de la Dermatite Atopique (DA). Ce modèle d'épiderme tridimensionnel présente également toutes les caractéristiques d'une fonction barrière altérée. Ce modèle est obtenu en stimulant un substitut cutané avec un réactif pro-inflammatoire contenant au moins de l'IL-1β. Ce réactif induit la production de médiateurs moléculaires clés dans la pathologie de la DA et une modulation de la fonction barrière cutanée. La présente invention concerne également une méthode de criblage ou d'évaluation de principes actifs pouvant avantageusement être utilisés dans le traitement de la DA et des peaux sèches à très sèches à tendance atopique.

### Description de l'art antérieur

La DA est définie, en clinique, comme une maladie inflammatoire de la peau ayant une grande incidence dans les pays industrialisés. Elle est caractérisée par un terrain génétique complexe, un système immunitaire perturbé, l'implication de facteurs environnementaux et des manifestations typiques sur la peau (Wollenberg, A., Rawer, H. C. & Schauber, J. (2011) Innate immunity in atopic dermatitis. Clin.Rev.Allergy Immunol., 41, 272-281). La DA implique également un défaut de barrière cutanée. La filaggrine est une des protéines importantes de la différenciation terminale des kératinocytes et donc de la fonction barrière de la peau. Les mutations non sens du gène codant pour cette protéine représentent un facteur de prédisposition majeur de la DA, de l'asthme et des allergies (Cornelissen, C., Marquardt, Y., Czaja, K., Wenzel, J., Frank, J., Luscher-Firzlaff, J., Luscher, B. & Baron, J. M. (2011) IL-31 regulates differentiation and filaggrin expression in human organotypic skin models. J.Allergy Clin.Immunol.)*.* Très récemment, une étude a permis de montrer que, dans la peau des patients DA, la filaggrine est moins exprimée que chez des sujets sains quelque soit le génotype (Pellerin, L., Henry, J., Hsu, C. Y., Balica, S., Jean-Decoster, C., Mechin, M. C., Hansmann, B., Rodriguez, E., Weindinger, S., Schmitt, A. M., Serre, G., Paul, C. & Simon, M. (2013) Defects of filaggrin-like proteins in both lesional and nonlesional atopic skin. J.Allergy Clin.Immunol., 131, 1094-1102). Par ailleurs, une forte diminution de la claudine-1, protéine des jonctions serrées, a été observée dans la peau des patients atteints de DA (De Benedetto, A., Rafaels, N. M., McGirt, L. Y., Ivanov, A. I., Georas, S. N., Cheadle, C., Berger, A. E., Zhang, K., Vidyasagar, S., Yoshida, T., Boguniewicz, M., Hata, T., Schneider, L. C., Hanifin, J. M., Gallo, R. L., Novak, N., Weidinger, S., Beaty, T. H., Leung, D. Y., Barnes, K. C. & Beck, L. A. (2011) Tight junction defects in patients with atopic dermatitis. J.Allergy Clin.Immunol., 127, 773-786). Outre une altération de ces protéines structurales essentielles au maintien de l'homéostasie épidermique, une déficience dans la production des peptides anti-microbiens (PAMs) ou un défaut d'expression du récepteur Toll-like de type 2 (TLR-2) qui assurent ensemble l'immunité innée épithéliale, ont été aussi observés dans la peau de patients DA (Niebuhr, M., Heratizadeh, A., Wichmann, K., Satzger, I. & Werfel, T. (2011) Intrinsic alterations of pro-inflammatory mediators in unstimulated and TLR-2 stimulated keratinocytes from atopic dermatitis patients. Exp.Dermatol., 20, 468-472).

Les modèles *in vitro* de DA sont un champ d'investigation permanent. Quelques modèles *in vitro* de DA ont déjà été décrits dans l'art antérieur:
- un modèle de kératinocytes cultivés en monocouche (Kinoshita, H., Takai, T., Le, T. A., Kamijo, S., Wang, X. L., Ushio, H., Hara, M., Kawasaki, J., Vu, A. T., Ogawa, T., Gunawan, H., Ikeda, S., Okumura, K. & Ogawa, H. (2009) Cytokine milieu modulates release of thymic stromal lymphopoietin from human keratinocytes stimulated with double-stranded RNA. J.Allergy Clin.Immunol., 123, 179-186) : sur ces cellules, Kinoshita démontre l'induction de TSLP par la combinaison de Poly I:C et TNFα ou la combinaison Poly I:C et IL-4 + IL13 (Kinoshita, H., Takai, T., Le, T. A., Kamijo, S., Wang, X. L., Ushio, H., Hara, M., Kawasaki, J., Vu, A. T., Ogawa, T., Gunawan, H., Ikeda, S., Okumura, K. & Ogawa, H. (2009) Cytokine milieu modulates release of thymic stromal lymphopoietin from human keratinocytes stimulated with double-stranded RNA. J.Allergy Clin.Immunol., 123, 179-186). Ce modèle de kératinocytes cultivés en monocouche présente cependant l'inconvénient de travailler sur des cellules prolifératives et non différenciées. De ce fait, elles n'expriment pas la plupart des marqueurs de différenciation épidermique. Le modèle est donc éloigné de la pathologie DA qui affecte toutes les couches vivantes de l'épiderme, dont une des couches les plus différenciées de l'épiderme, la couche granuleuse. Cette dernière est essentielle à la formation des précurseurs de l'enveloppe cornée et du ciment lipidique qui sont à l'origine de la fonction barrière cutanée, essentiellement portée par la couche cornée, la couche la plus superficielle et la plus différenciée de l'épiderme. Par ailleurs, ce modèle de kératinocytes cultivés en monocouche est limitant par le fait qu'il ne permet pas de cribler des principes actifs à vocation de protection ou de réparation de la fonction barrière et de l'intégrité tissulaire. Il ne permet pas non plus de procéder à des évaluations de produits ou formulations par voie topique. Ce modèle n'est donc pas adapté pour l'évaluation de composés candidats au traitement de la DA ou à la réparation de la fonction barrière de la peau.
- un modèle sur explant de peau humaine : les auteurs ont observé une induction de TSLP par la combinaison des cytokines IL4 et IL13 (Bogiatzi, S. I., Fernandez, I., Bichet, J. C., Marloie-Provost, M. A., Volpe, E., Sastre, X. & Soumelis, V. (2007) Cutting Edge: Proinflammatory and Th2 cytokines synergize to induce thymic stromal lymphopoietin production by human skin keratinocytes. J.Immunol., 178, 3373-3377), ou par la combinaison TNFα + IL4 (Bogiatzi, S. I., Guillot-Delost, M., Cappuccio, A., Bichet, J. C., Chouchane-Mlik, O., Donnadieu, M. H., Barillot, E., Hupe, P., Chlichlia, K., Efremidou, E. I., Aractingi, S., Bayrou, O. & Soumelis, V. (2012) Multiple-checkpoint inhibition of thymic stromal lymphopoietin-induced T(H)2 response by T(H)17-related cytokines. J.Allergy Clin.Immunol.). Un tel modèle présente l'inconvénient de dépendre de déchets opératoires de peau humaine ce qui introduit une contrainte liée au calendrier des opérations. Le criblage de principes actifs et de formulations peut être limité par cette variable. En outre, une contrainte supplémentaire est liée à la grande variabilité inter-donneur qui nécessite des évaluations sur un grand nombre de donneurs.
- D'autres auteurs ont rapportés des modèles désignés comme « modèles de DA » mais sans démontrer la production de TSLP (Kamsteeg, M., Bergers, M., de Boer, R., Zeeuwen, P. L., Hato, S. V., Schalkwijk, J. & Tjabringa, G. S. (2011) Type 2 helper T-cell cytokines induce morphologic and molecular characteristics of atopic dermatitis in human skin equivalent. Am.J.Pathol., 178, 2091-2099); or le marqueur TSLP est essentiel dans l'initiation de la DA et doit être un marqueur indispensable à obtenir *in vitro.*

Il ressort de ce constat général qu'il est nécessaire de disposer d'un modèle tissulaire *in vitro* pertinent et robuste permettant d'identifier des composés modulateurs de la réponse inflammatoire induite. Ces composés pourront en effet être utilisés pour traiter la DA ou pour réparer la fonction barrière de la peau.

Ce modèle tissulaire pourra plus particulièrement être utilisé pour cribler des composés candidats et sélectionner ceux présentant un effet sur les différentes caractéristiques épidermiques de la DA, c'est à dire sur le défaut de barrière associé à une altération de l'intégrité structurale, sur la réponse innée épithéliale spécifique, et/ou sur la réponse inflammatoire kératinocytaire spécifique. Ce modèle tissulaire doit être pleinement différencié, reproductible et robuste, afin de permettre l'évaluation de compositions contenant lesdits composés candidats et destinées à être utilisées sous forme topique.

### Description détaillée de l'invention

La cytokine TSLP (« Thymic stromal lymphopoietin ») est reconnue par l'homme de l'art comme le marqueur clé dans l'initiation de la DA. Soumelis et al, ont montré que TSLP était fortement exprimée par les kératinocytes des patients atteints de DA. Cette cytokine est associée au recrutement et à l'activation des cellules de Langerhans *in situ,* ce qui a permis de suggérer pour la première fois que TSLP pouvait être une cytokine initiatrice précoce et directe des cellules dendritiques (Soumelis, V., Reche, P. A., Kanzler, H., Yuan, W., Edward, G., Homey, B., Gilliet, M., Ho, S., Antonenko, S., Lauerma, A., Smith, K., Gorman, D., Zurawski, S., Abrams, J., Menon, S., McClanahan, T., Waal-Malefyt, R. R., Bazan, F., Kastelein, R. A. & Liu, Y. J. (2002) Human epithelial cells trigger dendritic cell mediated allergic inflammation by producing TSLP. Nat.Immunol., 3, 673-680). De plus, il a été démontré que TSLP est un élément essentiel dans le processus d'inflammation allergique chez l'homme (Liu, Y. J. (2006) Thymic stromal lymphopoietin: master switch for allergic inflammation. J.Exp.Med., vol 203 N°2, 269-273) (Takai, T. (2012) TSLP expression: cellular sources, triggers, and regulatory mechanisms. Allergol.Int., 61, 3-17) impliqué dans la physiopathologie de la DA mais aussi de l'asthme (Shikotra, A., Choy, D. F., Ohri, C. M., Doran, E., Butler, C., Hargadon, B., Shelley, M., Abbas, A. R., Austin, C. D., Jackman, J., Wu, L. C., Heaney, L. G., Arron, J. R. & Bradding, P. (2012) Increased expression of immunoreactive thymic stromal lymphopoietin in patients with severe asthma. J.Allergy Clin.Immunol., 129, 104-111), de l'oesophagite à éosinophiles (Sherrill, J. D., Gao, P. S., Stucke, E. M., Blanchard, C., Collins, M. H., Putnam, P. E., Franciosi, J. P., Kushner, J. P., Abonia, J. P., Assa'ad, A. H., Kovacic, M. B., Biagini Myers, J. M., Bochner, B. S., He, H., Hershey, G. K., Martin, L. J. & Rothenberg, M. E. (2010) Variants of thymic stromal lymphopoietin and its receptor associate with eosinophilic esophagitis. J.Allergy Clin.Immunol., 126, 160-165) et de la rhinite allergique (Bunyavanich, S., Melen, E., Wilk, J. B., Granada, M., Soto-Quiros, M. E., Avila, L., Lasky-Su, J., Hunninghake, G. M., Wickman, M., Pershagen, G., O'Connor, G. T., Weiss, S. T. & Celedon, J. C. (2011) Thymic stromal lymphopoietin (TSLP) is associated with allergic rhinitis in children with asthma. Clin.Mol.Allergy., 9:1).

Outre TSLP, d'autres cytokines et chemokines sont considérées comme des marqueurs de cette pathologie. Ainsi, les concentrations en RANTES/CCL5 et IL-6 dans les sérums de patient DA sont directement corrélées avec la sévérité de la maladie (Homey, B., Steinhoff, M., Ruzicka, T. & Leung, D. Y. (2006) Cytokines and chemokines orchestrate atopic skin inflammation. J.Allergy Clin.Immunol., 118, 178-189; Samochocki, Z., Alifier, M., Bodera, P., Jeziorkowska, R., Rosiak, E., Jurkiewicz, B., Glinska, O., Glinski, W. & Stankiewicz, W. (2012) T-regulatory cells in severe atopic dermatitis: alterations related to cytokines and other lymphocyte subpopulations. Arch.Dermatol.Res., 304, 795-801). De même, l'expression de MCP-3/CCL7 semble augmentée dans les zones tissulaires préalablement challengées de patients DA (Ying, S., Robinson, D. S., Meng, Q., Barata, L. T., McEuen, A. R., Buckley, M. G., Walls, A. F., Askenase, P. W. & Kay, A. B. (1999) C-C chemokines in allergen-induced late-phase cutaneous responses in atopic subjects: association of eotaxin with early 6-hour eosinophils, and of eotaxin-2 and monocyte chemoattractant protein-4 with the later 24-hour tissue eosinophilia, and relationship to basophils and other C-C chemokines (monocyte chemoattractant protein-3 and RANTES). J.Immunol., 163, 3976-3984). Enfin, le taux d'IL-8 dans le stratum corneum de la peau de patients DA est également corrélé avec la sévérité de la maladie dans l'étude d'Amarbayasgalan et al (Amarbayasgalan, T., Takahashi, H., Dekio, I. & Morita, E. (2012) Interleukin-8 Content in the Stratum Corneum as an Indicator of the Severity of Inflammation in the Lesions of Atopic Dermatitis. Int.Arch.Allergy Immunol., 160, 63-74). Xie et al (Journal of Dermatological Science 66 (2012) 233-237) décrit que la TSLP est fortement exprimée dans les kératinocytes des patients atteints de la dermatite atopique. Ce document décrit aussi que dans une culture de kératinocytes humains les ligands TLRs induisent l'expression de la TSLP : le poly I:C (TLR3), le lipopeptide FSL-1 diacylé (TLR2-TLR6).

Le but des présents inventeurs étaient de disposer d'un modèle épidermique tridimensionnel stratifié et différencié permettant de déterminer *in vitro,* de façon fiable et reproductible, la capacité d'un composé (ou d'une composition le contenant) à moduler les marqueurs de la DA, et notamment la sécrétion de la cytokine TSLP, ainsi qu'éventuellement la sécrétion des autres cytokines décrites ci-dessus.

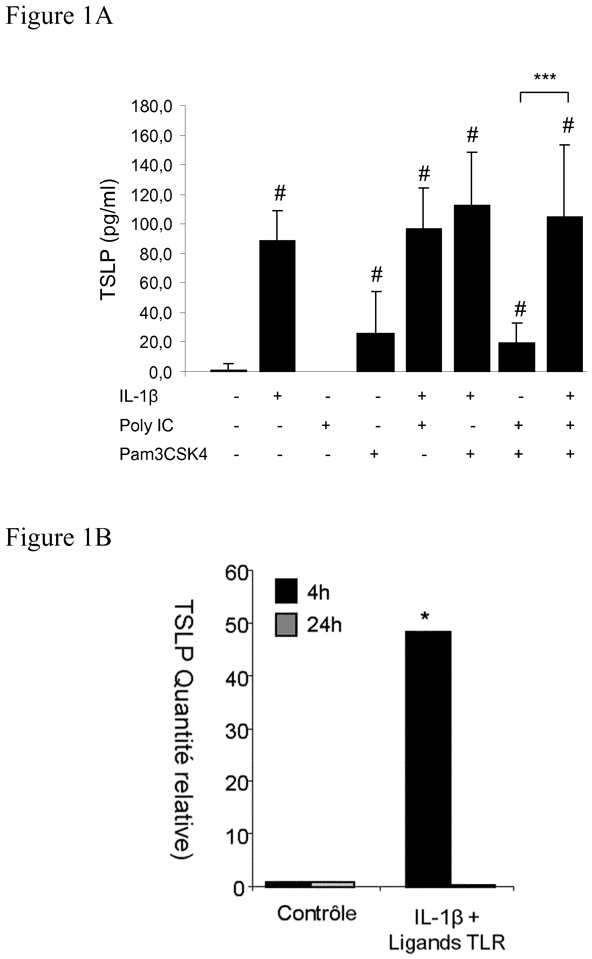

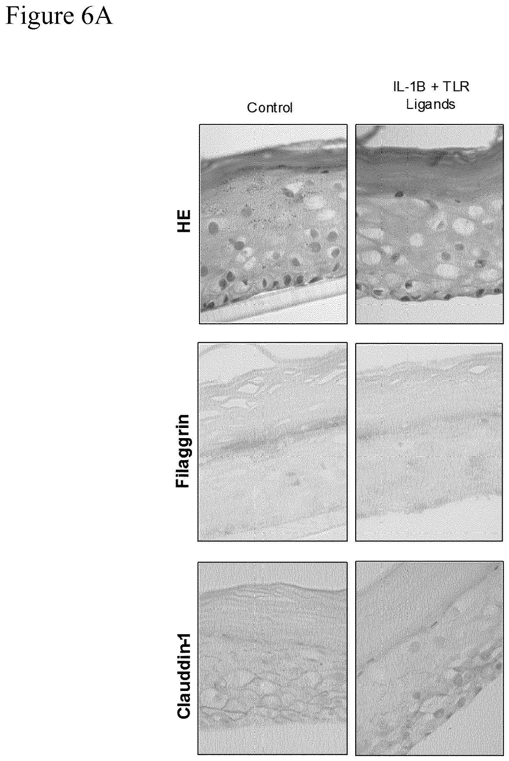

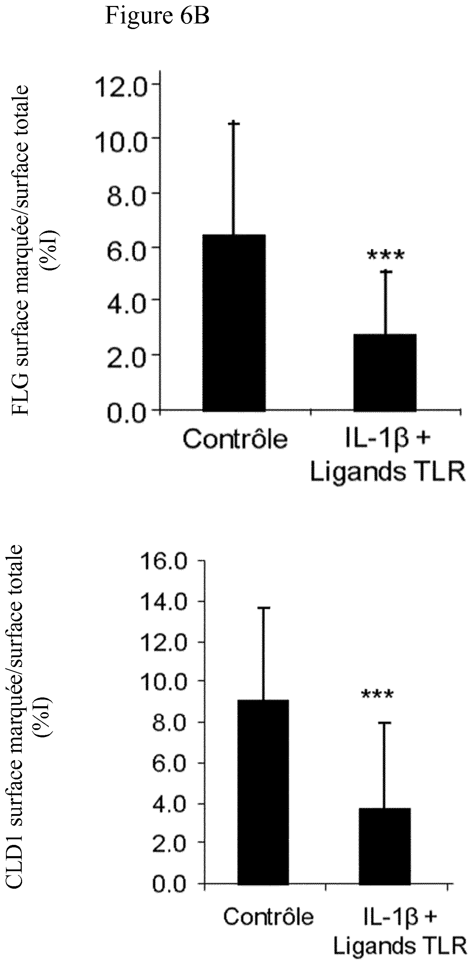

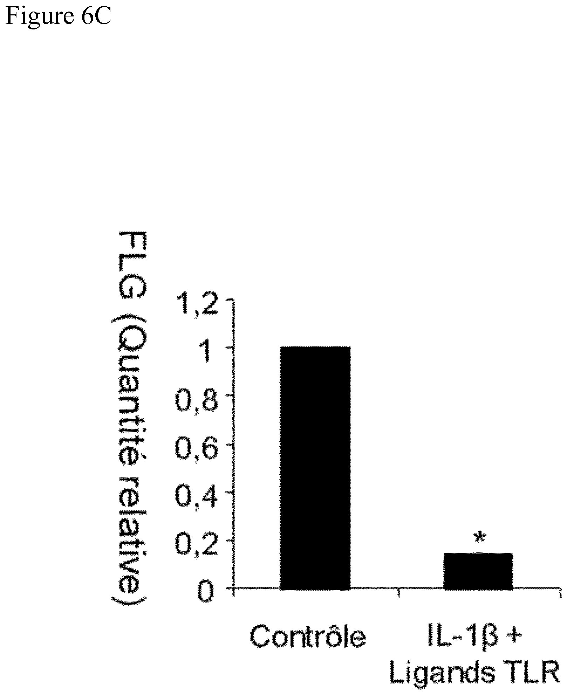

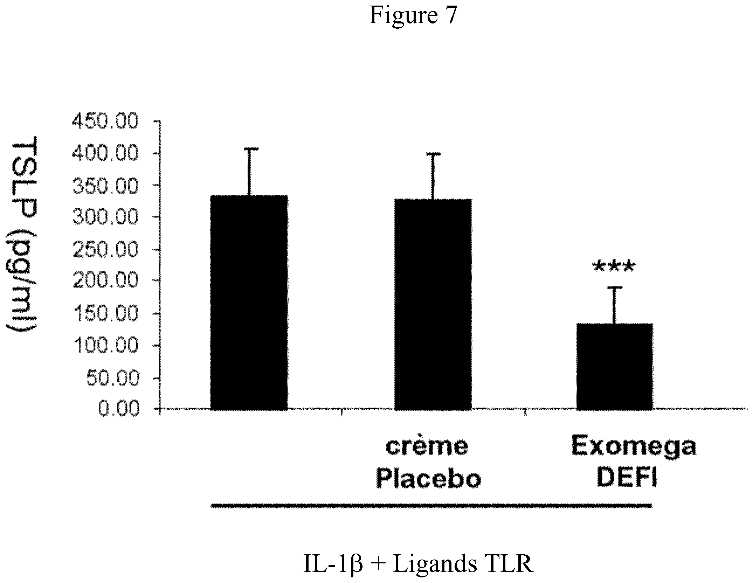

Dans ce contexte, les inventeurs ont identifié une combinaison pro-inflammatoire apte à générer la production de ces marqueurs de manière significative et reproductible. En particulier, cette combinaison permet d'induire, dans un explant cutané, une peau reconstruite ou dans un épiderme reconstruit:
- la production de TSLP de manière significative et reproductible (cf. figures 1A et 7);
- la production d'autres marqueurs clés de la DA tels que RANTES/CCL5 (figure 2A) et/ou MCP-3/CCL7 (figure 3A) et/ou IL-6 (figure 4A) et/ou IL-8 (figure 5A) de manière significative et reproductible;
- la génération d'une signature transcriptionnelle caractéristique de celle décrite dans l'épiderme de patient DA (cf. figures 1B, 2B, 3B, 4B, et 5B) ; et
- la dégradation de marqueurs ultrastructuraux indicateurs de l'intégrité de la barrière cutanée telle que celle observée *in vivo* dans la peau de patients DA (figure 6).

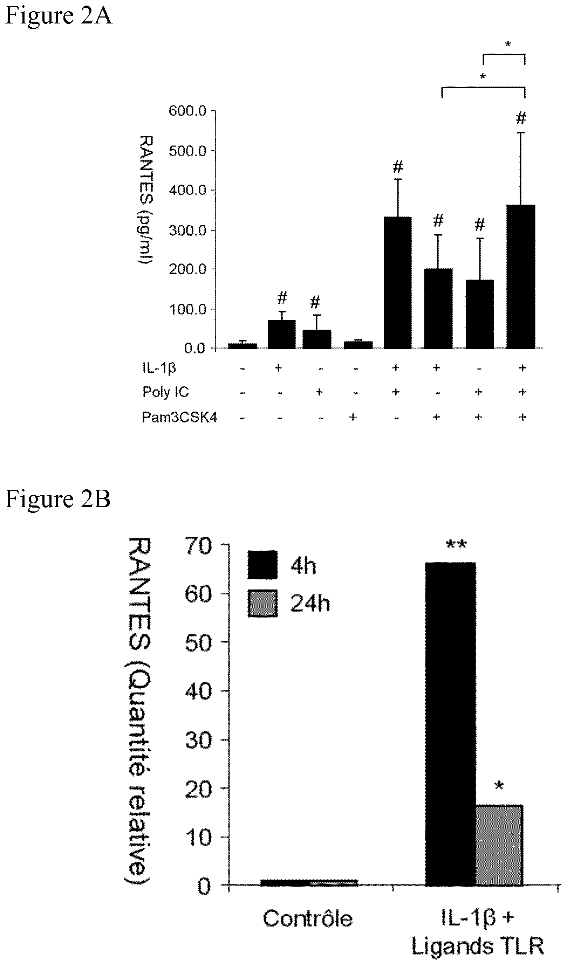

Une caractéristique essentielle de la combinaison pro-inflammatoire selon la présente invention est qu'elle contient de l'interleukine IL-1β. Les inventeurs ont en effet pu démontrer que l'incubation d'une peau ou d'un épiderme reconstruit(e) avec un réactif contenant une quantité efficace d'IL-1β induisait une forte production de TSLP (cf. figure 1), et des autres marqueurs de la DA (cf. figures 2 à 5).

Les inventeurs ont également constaté qu'il était avantageux d'associer l'IL-1β à un ligand du récepteur TLR-2 (SEQ ID NO :9) et/ou un ligand du récepteur TLR-3 (SEQ ID NO :10).

Selon un premier aspect, la présente invention concerne donc l'utilisation, selon la revendication 1, d'un réactif de stimulation pro-inflammatoire contenant entre 10 et 500 ng/mL, de préférence entre 20 et 200 ng/mL, de préférence entre 50 et 200 ng/mL, de manière encore plus préférée, 60ng/mL d'interleukine IL-1β, pour reproduire *in vitro* la physiopathologie de la dermatite atopique et/ou altérer la fonction barrière dans une peau reconstruite ou un épiderme reconstruit qui contient des cellules différenciées réparties en plusieurs couches, ou dans un explant cutané ; ledit réactif de stimulation pro-inflammatoire comprenant en outre un ligand du récepteur TLR-2 choisi parmi un peptidoglycane, de l'acide lipoteichoique, une lipoprotéine, un lipoarabinomanne, et du zymosan, et/ou un ligand du récepteur TLR-3 choisi parmi le poly (A :U) et le poly I:C.

Dans un mode de réalisation particulier, ledit réactif contient en particulier :
a. Entre 3 et 20 µg/mL, de préférence entre 3 et 15 µg/mL, d'un ligand du récepteur TLR-2, ou
b. Entre 3 et 20 µg/mL, de préférence entre 3 et 15 µg/mL, d'un ligand du récepteur TLR-3.

Dans un mode de réalisation encore plus particulier, ledit réactif contient en particulier :
a. Entre 3 et 20 µg/mL, de préférence entre 3 et 15 µg/mL, d'un ligand du récepteur TLR-2, et
b. Entre 3 et 20 µg/mL, de préférence entre 3 et 15 µg/mL, d'un ligand du récepteur TLR-3.

L'IL-1β est une cytokine sécrétée par les macrophages, les monocytes, les cellules dendritiques et les kératinocytes. Elle augmente l'expression des facteurs d'adhésion sur les cellules endothéliales, afin de faciliter la transmigration des leucocytes sur le ou les sites d'infection. Cette cytokine a, chez l'homme, la SEQ ID NO:1. Elle est vendue sous forme recombinante par différentes sociétés, notamment Tebu-Bio, R&D systems et Invivogen.

Le TLR-2 (« Toll-like receptor 2 ») reconnaît de nombreux motifs antigéniques (PAMP pour « Pathogen Associated molecular patterns ») localisés à la surface de pathogènes (lipoprotéines de membranes bactériennes, peptidoglycanes de bactéries Gram+, zymosan, hémagglutinine du virus des oreillons,...). De préférence, dans le cadre de la présente invention, le ligand du récepteur TLR2 est un peptidoglycane, de l'acide lipoteichoique, une lipoprotéine, un lipoarabinomanne, ou du zymosan. Le ligand du récepteur TLR2 est en effet un composé de la paroi cellulaire d'une bactérie Gram-positive choisi parmi un peptidoglycane, l'acide lipoteichoique, une lipoprotéine, un lipoarabinomanne de mycobactérie et le zymosan de la paroi cellule de la levure. De manière encore plus préférée, dans le cadre de la présente invention, le ligand du récepteur TLR2 est le lipopeptide tri-acylé Pam3CSK4 de formule :

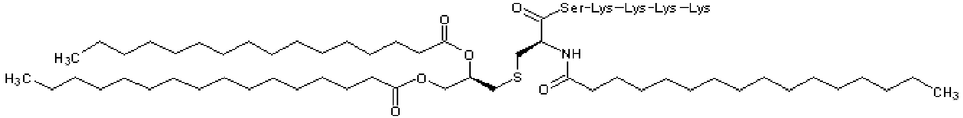

Ce ligand est commercialisé par différentes sociétés, notamment Invivogen, Tocris Bioscience.

Le TLR-3 (« Toll-like receptor 3 ») reconnait quant à lui exclusivement l'ARN double brin. Le Poly I :C (poly[(2R,3S,4R,5R)-5-(4-amino-2-oxopyrimidin-1-yl)-3,4-dihydroxyoxolan-2-yl]methyl dihydrogen phosphate), de formule : 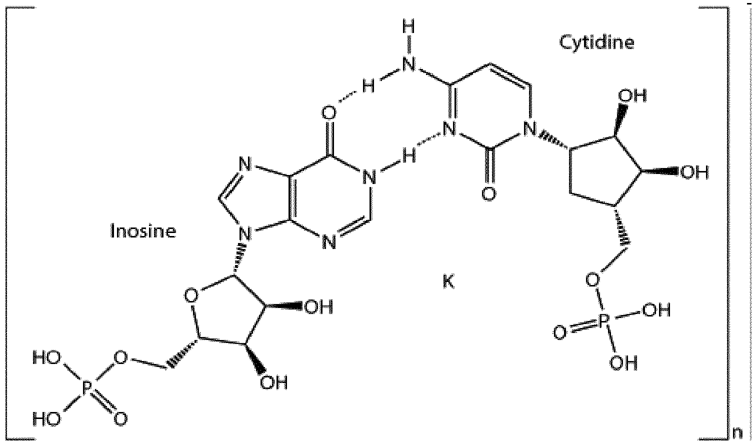 est le ligand le plus couramment utilisé pour le TLR3. Il est commercialisé par de nombreuses sociétés, notamment Invivogen, Tocris Bioscience. D'autres ligands du TLR3 peuvent être utilisés, comme le poly (A :U). Dans le cadre de la présente invention, le ligand du TLR3 utilisé est de préférence le poly (I :C).

La présente demande décrit aussi que le réactif de stimulation pro-inflammatoire contient:
- 60ng/mL d'IL-1β,
- 10µg/mL de Poly I:C, et
- 5ng/mL de Pam3CSK4

Dans un autre aspect, la présente description décrit l'utilisation d'un réactif de stimulation pro-inflammatoire contenant de l'interleukine IL-1β pour reproduire *in vitro* la physiopathologie de la DA et/ou altérer la fonction barrière dans un substitut cutané ou dans un explant cutané. Cette utilisation est de préférence réalisée sur un substitut cutané.

Dans cette utilisation, ledit réactif contient entre 10 et 500 ng/mL, de préférence entre 20 et 200 ng/mL, de manière encore plus préférée, 60ng/mL d'interleukine IL-1β.

Dans un mode de réalisation préféré, ledit réactif pro-inflammatoire comprend en outre un ligand du récepteur TLR-2 et/ou un ligand du récepteur TLR-3, lesdits ligands étant tels que définis ci-dessus. Dans un mode de réalisation encore plus préféré, ledit ligand du récepteur TLR-2 est Pam3CSK4 et en ledit ligand du récepteur TLR-3 est le poly I:C.

Dans un mode de réalisation encore plus préféré, la présente invention vise l'utilisation d'un réactif de stimulation pro-inflammatoire contenant :
- entre 10 et 500 ng/mL, de préférence entre 20 et 200 ng/mL, de préférence entre 50 et 200 ng/mL, de manière encore plus préférée, 60ng/mL d'interleukine IL-1β, et
- Entre 3 et 20 µg/mL, de préférence entre 3 et 15 µg/mL, de manière encore plus préférée 5µg/mL de Pam3CSK4 ou
- Entre 3 et 20 µg/mL, de préférence entre 3 et 15 µg/mL, de manière encore plus préférée 10µg/mL de Poly (I :C).

En d'autres termes, la présente invention vise l'utilisation d'un réactif de stimulation pro-inflammatoire contenant :
- entre 10 et 500 ng/mL, de préférence entre 20 et 200 ng/mL, de préférence entre 50 et 200 ng/mL, de manière encore plus préférée, 60ng/mL d'interleukine IL-1β, et
- Entre 3 et 20 µg/mL, de préférence entre 3 et 15 µg/mL, de manière encore plus préférée 5µg/mL de Pam3CSK4
ou l'utilisation d'un réactif de stimulation pro-inflammatoire contenant :
- entre 10 et 500 ng/mL, de préférence entre 20 et 200 ng/mL, de préférence entre 50 et 200 ng/mL, de manière encore plus préférée, 60ng/mL d'interleukine IL-1β, et
- Entre 3 et 20 µg/mL, de préférence entre 3 et 15 µg/mL, de manière encore plus préférée 10µg/mL de Poly (I :C).

Dans un mode de réalisation préféré entre tous, la présente invention vise l'utilisation d'un réactif de stimulation pro-inflammatoire contenant :
- entre 10 et 500 ng/mL, de préférence entre 20 et 200 ng/mL, de préférence entre 50 et 200 ng/mL, de manière encore plus préférée, 60ng/mL d'interleukine IL-1β, et
- Entre 3 et 20 µg/mL, de préférence entre 3 et 15 µg/mL, de manière encore plus préférée 5µg/mL de Pam3CSK4 et
- Entre 3 et 20 µg/mL, de préférence entre 3 et 15 µg/mL, de manière encore plus préférée 10µg/mL de Poly (I :C).

Ainsi, le réactif de stimulation pro-inflammatoire peut contenir avantageusement:
- 60ng/mL d'IL-1β,
- 10µg/mL de Poly I:C, et
- 5µg/mL de Pam3CSK4

Dans le contexte de la présente invention, le terme « explant cutané » correspond à une biopsie de peau humaine issue de déchet opératoire et qui comprend la totalité de l'épiderme et un compartiment dermique.

Le terme « substitut cutané » désigne par ailleurs dans la présente invention une peau reconstruite ou un épiderme reconstruit, de préférence humain, qui contient des cellules différenciées, réparties en plusieurs couches. En particulier, la peau reconstruite contient, au sens de la présente invention, au moins deux compartiments, un compartiment dermique, et un compartiment épidermique.

Par « épiderme reconstruit », on entend au sens de la présente invention un épiderme généré *in vitro* par des techniques classiques bien connues de l'homme du métier (cf., par exemple, Use of Epidermal Equivalents Generated from Follicular Outer Root Sheath Cells in vitro and for Autologous Grafting of Chronic Wounds. Alain Limat, Thomas Hunziger. Cells Tissues Organs 2002;172:79-85 ; A simple reconstructed human epidermis: preparation of the culture model and utilization in in vitro studies) (Poumay Y, Dupont F, Marcoux S, Leclercq-Smekens M, Hérin M, Coquette A. Arch Dermatol Res. 2004 Oct;296(5):203-11. Epub 2004 Sep 2.). Les kératinocytes utilisés dans cet épiderme peuvent être issus de peau humaine saine ou de peau sèche à très sèche, ou de peau de patients atteints de dermatite atopique, d'ichtyoses héréditaires, ou de psoriasis.

Par « peau reconstruite », on entend au sens de la présente invention une composante épidermique contenant des kératinocytes, générée *in vitro* et une composante dermique contenant des fibroblastes, générée *in vitro.* La peau reconstruite est disponible commercialement. Les kératinocytes et les fibroblastes utilisés dans cette peau peuvent être issus de peau humaine saine ou de peau humaine sèche à très sèche, ou de peau de patients atteints de dermatite atopique, d'ichtyoses héréditaires, ou de psoriasis.

Par « peau humaine », on entend au sens large une peau humaine saine ou une peau sèche à très sèche, ou encore une peau provenant de patients atteints de pathologies cutanées associées à une perte de l'homéostasie cutanée telles que la dermatite atopique, des ichtyoses héréditaires, le psoriasis.

Ainsi, le modèle de le présente description est un modèle tridimensionnel d'explant cutané, de peau ou d'épiderme reconstruit, stratifié et différencié, qui présente des caractéristiques clés de la physiopathologie de la DA ou d'une fonction barrière altérée. Dans ce cas, les 4 couches épidermiques (couche basale, couche spineuse, couche granuleuse et couche cornée) y sont distinctes et fonctionnelles.

Par « fonction barrière de la peau », on entend, au sens de la présente invention, la protection conférée par la couche épidermique sur la peau. Cette fonction consiste plus particulièrement à protéger l'organisme de la déshydratation dans un milieu non aquatique. Pour mesurer la fonction et l'intégrité de la barrière cutanée en clinique, les méthodes biométriques sont utilisées. Les deux analyses fonctionnelles les plus courantes sont la perte insensible en eau, dont l'augmentation signifie une perte de la fonction de barrière, et la cornéométrie qui quantifie l'état d'hydratation de la couche cornée.

Par « fonction barrière altérée », on entend, au sens de la présente invention, une diminution de la fonction de protection telle que définie ci-dessus. Elle se traduit notamment par une sécheresse cutanée qui peut conduire à des pathologies secondaires comme les eczémas, les hypodermites, et les ulcères chroniques.

La présente invention vise également un procédé pour reproduire *in vitro* la physiopathologie de la DA et/ou altérer la fonction barrière dans une peau reconstruite ou un épiderme reconstruit qui contient des cellules différenciées réparties en plusieurs couches, ou un explant cutané, ladite méthode contenant les étapes :
i. Mettre en contact ledit substitut cutané ou ledit explant cutané avec un milieu de maintenance contenant un réactif de stimulation pro-inflammatoire contenant entre 10 et 500 ng/mL d'interleukine IL-1β, ledit milieu de maintenance contenant en outre entre 3 et 20 µg/mL d'un ligand du récepteur TLR-2, et/ou entre 3 et 20 µg/mL d'un ligand du récepteur TLR-3 et
ii. Incuber pendant 2 à 48 heures.

Ce procédé est de préférence mis en œuvre sur un substitut cutané, c'est-à-dire sur une peau reconstruite ou un épiderme reconstruit.

Le milieu de maintenance contient ainsi, outre de l'IL-1β, entre 3 et 20 µg/mL d'un ligand du récepteur TLR-2, et/ou entre 3 et 20 µg/mL d'un ligand du récepteur TLR-3, lesdits ligands étant tels que définis ci-dessus. Dans un mode de réalisation encore plus préféré, ledit ligand du récepteur TLR-2 est Pam3CSK4 et en ledit ligand du récepteur TLR-3 est le poly I:C.

Dans un mode de réalisation encore plus préféré, ledit milieu de maintenance contient:
- entre 10 et 500 ng/mL, de préférence entre 20 et 200 ng/mL, de manière encore plus préférée, 60ng/mL d'interleukine IL-1β, et
- Entre 3 et 20 µg/mL, de préférence entre 3 et 15 µg/mL, de manière encore plus préférée 5µg/mL de Pam3CSK4 ou
- Entre 3 et 20 µg/mL, de préférence entre 3 et 15 µg/mL, de manière encore plus préférée 10µg/mL de Poly (I :C).

Dans un mode de réalisation préféré entre tous, ledit milieu de maintenance contient :
- entre 10 et 500 ng/mL, de préférence entre 20 et 200 ng/mL, de manière encore plus préférée, 60ng/mL d'interleukine IL-1β, et
- Entre 3 et 20 µg/mL, de préférence entre 3 et 15 µg/mL, de manière encore plus préférée 5µg/mL de Pam3CSK4 et
- Entre 3 et 20 µg/mL, de préférence entre 3 et 15 µg/mL, de manière encore plus préférée 10µg/mL de Poly (I :C).

Ainsi, ledit milieu de maintenance peut contenir avantageusement:
- 60ng/mL d'IL-1β,
- 10µg/mL de Poly I:C, et
- 5µg/mL de Pam3CSK4

L'incubation peut être avantageusement réalisée à une température comprise entre 36°C et 38°C. Le taux de CO2 est généralement compris entre 4% et 6%. Enfin, cette incubation est préférentiellement réalisée dans une atmosphère saturée en humidité.

Dans un mode de réalisation particulier, on pourra consécutivement appliquer un stress de déshydratation (température, hygrométrie) et/ou un stress physique (UV).

Une incubation de quelques heures (typiquement entre 2 et 4 heures) du substitut cutané ou de l'explant cutané avec le réactif pro-inflammatoire de l'invention suffit pour induire la production des cytokines TSLP, RANTES, MCP-3, IL-6 et/ou IL-8, ce qui est observé en analysant le taux d'ARNm de ces cytokines (cf. figures 1B, 2B, 3B, 4B, et 5B). La production et la sécrétion de ces protéines est quant à elle observée après une incubation plus longue, typiquement entre 10 et 48h. Le temps d'incubation dépend donc de la mesure que l'on souhaite effectuer sur le modèle cutané.

Dans un autre aspect, la présente invention vise spécifiquement une peau reconstuite ou épiderme reconstruit qui contient des cellules différenciées réparties en plusieurs couches, ou explant cutané obtenu par le procédé décrit ci-dessus et reproduisant la physiopathologie de la dermatite atopique, ladite peau reconstruite ou ledit épiderme reconstruit sécrétant la cytokine TSLP. D'autre part, ils présentent une fonction barrière altérée.

Dans un mode de réalisation particulier, les cellules présentes dans ce substitut cutané ou cet explant cutané sécrètent la cytokine TSLP de SEQ ID NO :2. Dans un mode de réalisation préféré, les cellules présentes dans ce substitut cutané ou cet explant cutané sécrètent, outre la cytokine TSLP, la cytokine RANTES (SEQ ID NO :3) et/ou MCP-3 (SEQ ID NO :4) et/ou IL-6 (SEQ ID NO :5) et/ou IL-8 (SEQ ID NO :6). Dans un mode de réalisation encore plus préféré, les cellules présentes dans ce substitut cutané ou cet explant cutané sécrètent, outre la cytokine TSLP, les protéines RANTES (SEQ ID NO :3), MCP-3 (SEQ ID NO :4), IL-6 (SEQ ID NO :5) et IL-8 (SEQ ID NO :6). Un tel substitut cutané ou explant cutané sont idéaux pour étudier les mécanismes impliqués dans la physiopathologie de la DA et l'effet de composés modulateurs sur cette pathologie.

Selon la description de la présente démande, ce substitut cutané ou cet explant cutané présente une fonction barrière altérée et exprime les protéines filaggrine (SEQ ID NO :7) et claudine-1 (SEQ ID NO :8) en plus faible quantité par rapport à un épiderme ou une peau normale.

Dans un dernier aspect, la présente invention concerne une méthode de criblage selon la revendication 6 pour l'identification *in vitro* de composés destinés au traitement de la DA et des peaux sèches à tendance atopique, comprenant la mise en contact, par application topique ou par ajout dans le milieu de culture, desdits composés (ou de compositions les contenant) avec la peau reconstruite, l'épiderme reconstruit ou l'explant cutané de l'invention.

Ces composés peuvent être avantageusement inclus dans une composition, par exemple dans une composition cosmétique ou dermatologique (cf. exemple II ci-dessous).

Par « traitement de la DA », on fait référence ici de préférence au traitement d'appoint de cette pathologie (entre les crises).

Cette méthode de criblage comprend les étapes :
a. Mesurer la quantité de TSLP sécrétée,
b. Comparer la valeur obtenue à l'étape a) avec celles mesurées en l'absence du composé candidat.

La méthode est ainsi mise en œuvre sur un substitut cutané, c'est-à-dire sur une peau reconstruite ou un épiderme reconstruit.

De façon optionnelle, il est également possible de mesurer, outre la quantité de TSLP sécrétée, la quantité des protéines RANTES, MCP-3, IL-6 et IL-8 sécrétées, ainsi que l'expression des protéines filaggrine et claudine-1 dans le substitut cutané ou dans l'explant cutané.

Le composé candidat (ou une composition le contenant) sera sélectionné si la quantité de TSLP, et optionnellement de RANTES, de MCP-3, d'IL-6 et/ou d'IL-8 sécrétées diminue significativement selon un test statistique (par exemple : test de Student si les résultats suivent une loi normale ou test Mann Withney dans le cas contraire), et/ou si l'expression des protéines filaggrine et/ou claudine-1 augmente significativement selon un test statistique (par exemple : test de Student si les résultats suivent une loi normale ou test Mann Withney dans le cas contraire) dans le substitut cutané ou dans l'explant cutané lorsque le composé est ajouté.

La description divulgue aussi une méthode de criblage pour l'identification *in vitro* de composés destiné à restaurer la fonction barrière de la peau, comprenant la mise en contact, par application topique ou par ajout dans le milieu de culture, desdits composés (ou des compositions les contenant) avec le substitut cutané ou l'explant cutané de l'invention.

Cette méthode de criblage comprend de préférence les étapes :
a. Mesurer l'expression des protéines filaggrine et/ou claudine-1 dans ledit substitut cutané ou dans ledit explant cutané,
b. Comparer la valeur obtenue à l'étape a) avec celle(s) mesurée(s) en l'absence du composé candidat.

De façon optionnelle, il est également possible de mesurer, outre la quantité des protéines filaggrine et/ou claudine-1 exprimées, la quantité des protéines TSLP, RANTES, MCP-3, IL-6 et/ou IL-8 sécrétées par ledit substitut cutané ou par ledit explant cutané, avant et après l'application du composé candidat.

Un composé candidat (ou une composition le contenant) sera sélectionné si l'expression de la filaggrine et/ou de la claudine-1 mesurée après l'application de ce composé est supérieure à l'expression de la filaggrine et/ou de la claudine-1 mesurée en l'absence de ce composé.

Le composé candidat (ou une composition le contenant) sera sélectionné si la quantité de TSLP, et optionnellement de RANTES, de MCP-3, d'IL-6 et/ou d'IL-8 sécrétées diminue significativement selon un test statistique (par exemple : test de Student si les résultats suivent une loi normale ou test Mann Withney dans le cas contraire), et/ou si l'expression des protéines filaggrine et/ou claudine-1 augmente significativement selon un test statistique (par exemple : test de Student si les résultats suivent une loi normale ou test Mann Withney dans le cas contraire) dans le substitut cutané ou dans l'explant cutané lorsque le composé est ajouté.

La présente description divulgue enfin une méthode de criblage pour l'identification *in vitro* de composés ou compositions les contenant destinés à la prévention de la DA et des peaux sèches à tendance atopique, comprenant les étapes suivantes:
a. Appliquer un composé candidat ou une composition le contenant de façon topique sur un substitut cutané ou sur un explant cutané ou ajouter un composé candidat ou une composition le contenant dans leur milieu de culture,
b. Ajouter sur ledit substitut cutané ou sur ledit explant cutané un milieu de maintenance contenant un réactif de stimulation pro-inflammatoire contenant entre 10 et 500 ng/mL d'interleukine IL-1β,
c. Incuber pendant 2 à 48 heures,
d. Mesurer la quantité de TSLP sécrétée,
e. Comparer la valeur obtenue à l'étape d) avec celle mesurée en l'absence du composé candidat.

Cette méthode est mise en œuvre sur un substitut cutané, c'est-à-dire sur une peau reconstruite ou un épiderme reconstruit.

Dans un mode de réalisation préféré, ledit milieu de maintenance contient:
- entre 10 et 500 ng/mL, de préférence entre 20 et 200 ng/mL, de préférence entre 50 et 200 ng/mL, de manière encore plus préférée, 60ng/mL d'interleukine IL-1β, et
- Entre 3 et 20 µg/mL, de préférence entre 3 et 15 µg/mL, de manière encore plus préférée 5µg/mL de Pam3CSK4 ou
- Entre 3 et 20 µg/mL, de préférence entre 3 et 15 µg/mL, de manière encore plus préférée 10µg/mL de Poly (I :C).

Dans un mode de réalisation encore plus préféré, ledit milieu de maintenance contient :
- entre 10 et 500 ng/mL, de préférence entre 20 et 200 ng/mL, de préférence entre 50 et 200 ng/mL, de manière encore plus préférée, 60ng/mL d'interleukine IL-1β, et
- Entre 3 et 20 µg/mL, de préférence entre 3 et 15 µg/mL, de manière encore plus préférée 5ng/mL de Pam3CSK4 et
- Entre 3 et 20 µg/mL, de préférence entre 3 et 15 µg/mL, de manière encore plus préférée 10µg/mL de Poly (I :C).

Ainsi, ledit milieu de maintenance peut contenir avantageusement:
- 60ng/mL d'IL-1β,
- 10µg/mL de Poly I:C, et
- 5µg/mL de Pam3CSK4

De façon optionnelle, dans ce procédé, il est également possible de mesurer, outre la quantité de TSLP sécrétée, la quantité des protéines RANTES, MCP-3, IL-6 et IL-8 sécrétées, ainsi que l'expression des protéines filaggrine et claudine-1 dans le substitut cutané ou dans l'explant cutané.

Le composé candidat (ou une composition le contenant) sera sélectionné si la quantité de TSLP, et optionnellement de RANTES, de MCP-3, d'IL-6 et/ou d'IL-8 sécrétées diminue significativement selon un test statistique (par exemple : test de Student si les résultats suivent une loi normale ou test Mann Withney dans le cas contraire), et/ou si l'expression des protéines filaggrine et/ou claudine-1 augmente significativement selon un test statistique (par exemple : test de Student si les résultats suivent une loi normale ou test Mann Withney dans le cas contraire) dans le substitut cutané ou dans l'explant cutané lorsque le composé est ajouté.

Dans toutes ces méthodes de criblage, la quantité de protéine TSLP et des autres protéines sécrétées peut être mesurée par toute technique de dosage protéique connue de l'homme de l'art, et notamment par ELISA ou par western-blot. Par ailleurs, l'expression des protéines filaggrine et claudine-1 peut être évaluée en mesurant la quantité d'ARNm codant pour ces protéines, par qPCR, par Northern Blot ; ou par immunohistochimie sur des coupes de cellules.

L'incubation peut être avantageusement réalisée à une température comprise entre 36°C et 38°C. Le taux de CO2 est généralement compris entre 4% et 6%. Enfin, cette incubation est préférentiellement réalisée dans une atmosphère saturée en humidité.

Dans un mode de réalisation particulier, on pourra consécutivement appliquer un stress de déshydratation (température, hygrométrie) et/ou un stress physique (UV).

### Légendes des figures

La **figure 1** représente la production de TSLP par des épidermes reconstruits après différentes combinaisons stimulantes. (A) Analyse de la production de TSLP relarguée dans les sous-nageant de culture des épidermes reconstruits après 24 heures de stimulation. (B) Analyse à 4 heures et 24 heures de l'expression du gène TSLP par l'analyse en PCR quantitative en temps réel des ARNm extraits des épidermes reconstruits. #: P<0.05 versus control (Student's *t* test). *: P value (Student's *t* test) <0.05
La **figure 2** représente la production de la cytokine RANTES/CCL5 par des épidermes reconstruits après différentes combinaisons stimulantes. (A) Analyse de la production de RANTES/CCL5 relarguée dans les sous-nageant de culture des épidermes reconstruits après 24 heures de stimulation. (B) Analyse à 4 heures et 24 heures de l'expression des gènes codant pour RANTES/CCL5 par l'analyse en PCR quantitative en temps réel des ARNm extraits des épidermes reconstruits. #: P<0.05 versus control (Student's *t* test), *: P value (Student's *t* test) <0.05; **:P value (Student's *t* test) <0.01.

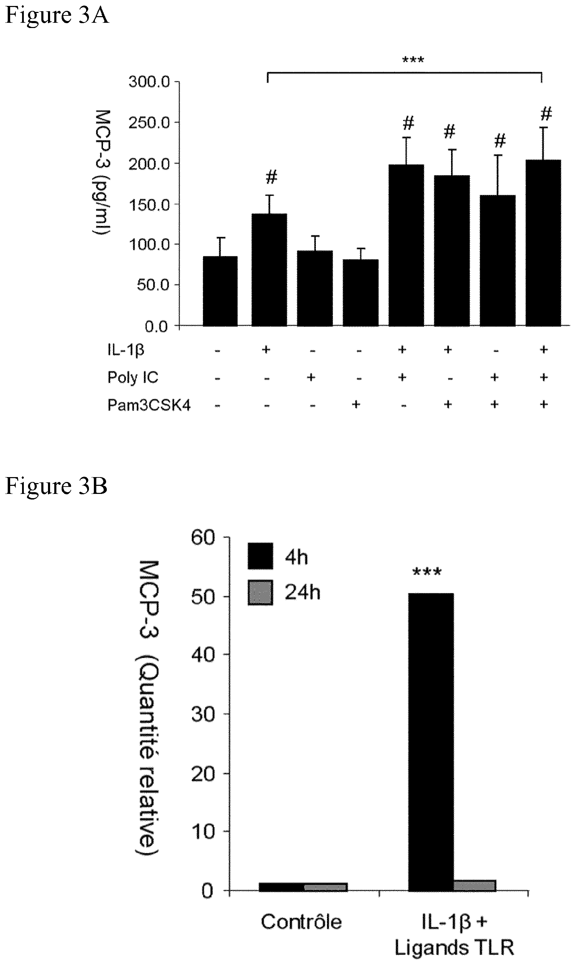

La **figure 3** représente la production de la cytokine MCP-3/CCL7 par des épidermes reconstruits après différentes combinaisons stimulantes. (A) Analyse de la production de MCP-3/CCL7 relarguée dans les sous-nageant de culture des épidermes reconstruits après 24 heures de stimulation. (B) Analyse à 4 heures et 24 heures de l'expression des gènes codant pour MCP-3/CCL7 par l'analyse en PCR quantitative en temps réel des ARNm extraits des épidermes reconstruits. #: P<0.05 versus control (Student's *t* test), *: P value (Student's *t* test) <0.05; **:P value (Student's *t* test) <0.01; ***: P value (Mann Withney test) <0.001.

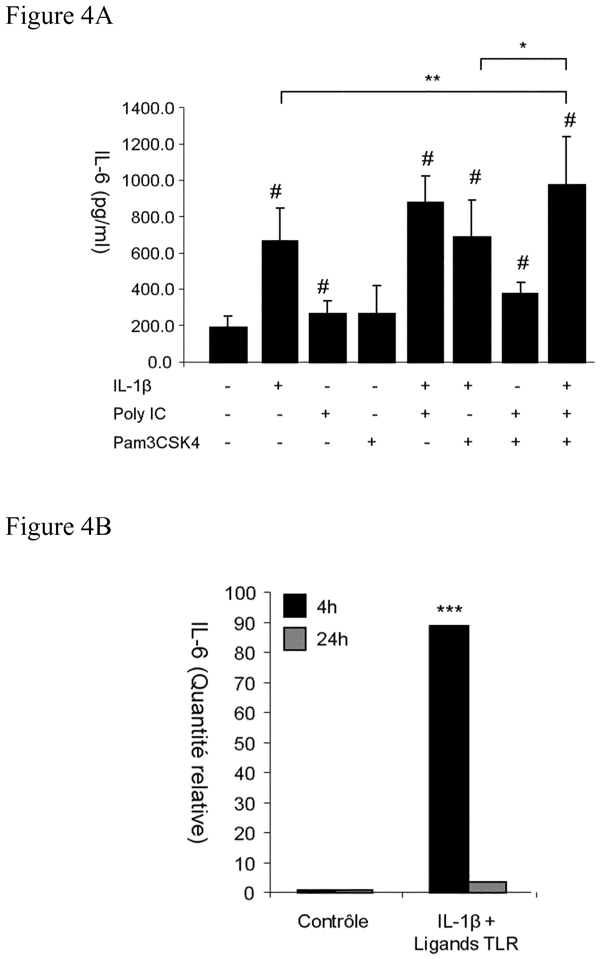

La **figure 4** représente la production de la cytokine IL-6 par des épidermes reconstruits après différentes combinaisons stimulantes. (A) Analyse de la production de IL-6 relarguée dans les sous-nageant de culture des épidermes reconstruits après 24 heures de stimulation. (B) Analyse à 4 heures et 24 heures de l'expression des gènes codant pour IL-6 par l'analyse en PCR quantitative en temps réel des ARNm extraits des épidermes reconstruits. #: P<0.05 versus control (Student's *t* test), *: P value (Student's *t* test) <0.05; **:P value (Student's *t* test) <0.01; ***: P value (Mann Withney test) <0.001.

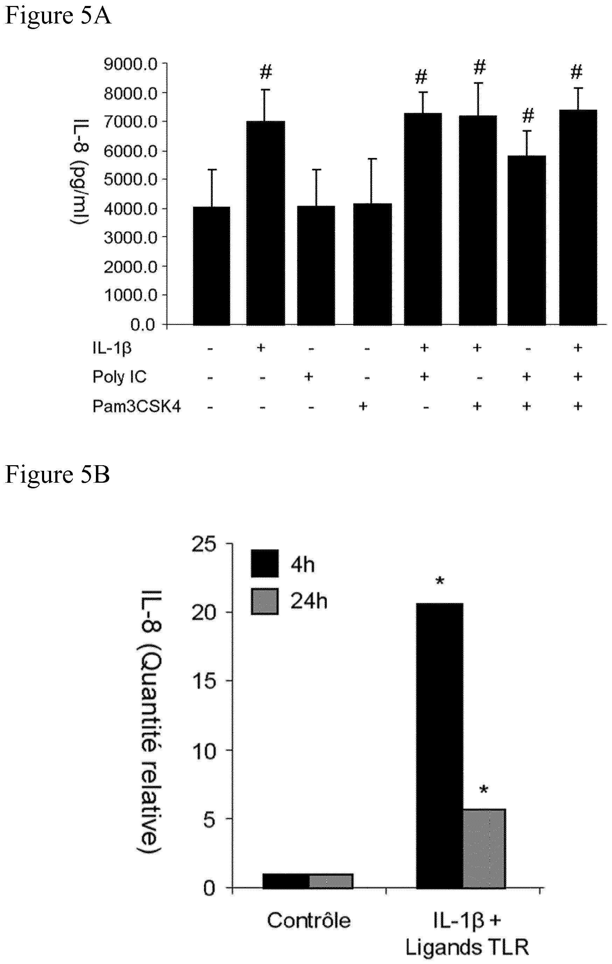

La **figure 5** représente la production de la cytokine IL-8 par des épidermes reconstruits après différentes combinaisons stimulantes. (A) Analyse de la production de IL-8 relarguée dans les sous-nageant de culture des épidermes reconstruits après 24 heures de stimulation. (B) Analyse à 4 heures et 24 heures de l'expression des gènes codant pour IL-8 par l'analyse en PCR quantitative en temps réel des ARNm extraits des épidermes reconstruits. #: P<0.05 versus control (Student's *t* test), *: P value (Student's *t* test) <0.05.
La **figure 6** concerne l'altération de l'intégrité épidermique et représente l'analyse immunohistologique des épidermes reconstruits après stimulation avec le milieu de maintenance préféré. (A) Coupes transversales d'épiderme reconstruit coloré à l'hématoxylin-eosine et Immunomarquages avec des anticorps spécifiques de la Filaggrine et de la Claudine-1 après 24 heures de stimulation. (B) : semi-quantification des immunomarquages correspondants. (C) Analyse de l'expression du gène codant pour la Filaggrine par l'analyse en PCR quantitative en temps réel des ARNm extraits des épidermes reconstruits. *: P value (Student's t test) <0.05, ***: P value (Mann Withney test) <0.001.
La **figure 7** représente l'inhibition de la production de TSLP médiée par la stimulation pro-inflammatoire par la crème A-Derma Exomega DEFI. *** P value (Student's test) < 0.001

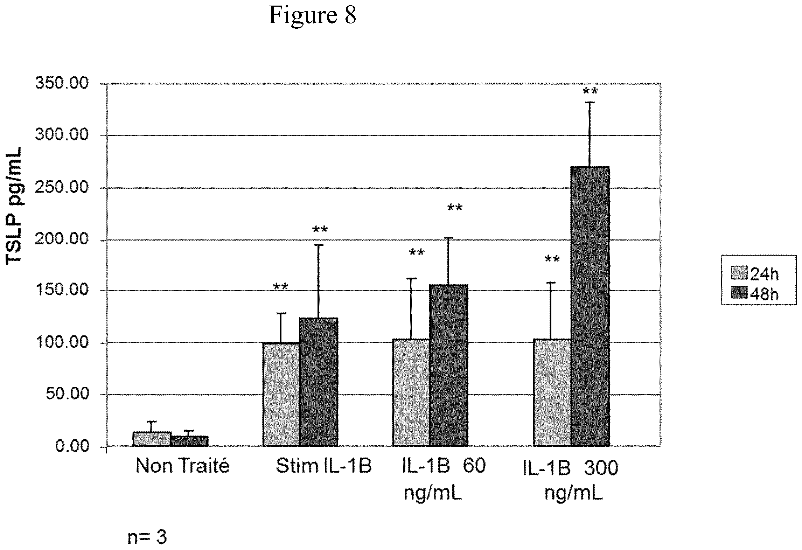

La **figure 8** représente la production de TSLP par des **explants cutanés** après différentes combinaisons stimulantes après 24 et 48 heures de stimulation. La combinaison Stim.IL-1B représente le réactif de stimulation contenant 60ng/mL d'IL-1β, 10µg/mL de Poly I:C, et 5µg/mL de Pam3CSK4 Une gamme de concentration avec le réactif IL-1β seul a été évaluée ** : p<0.01 versus non traité (test de Dunnett).

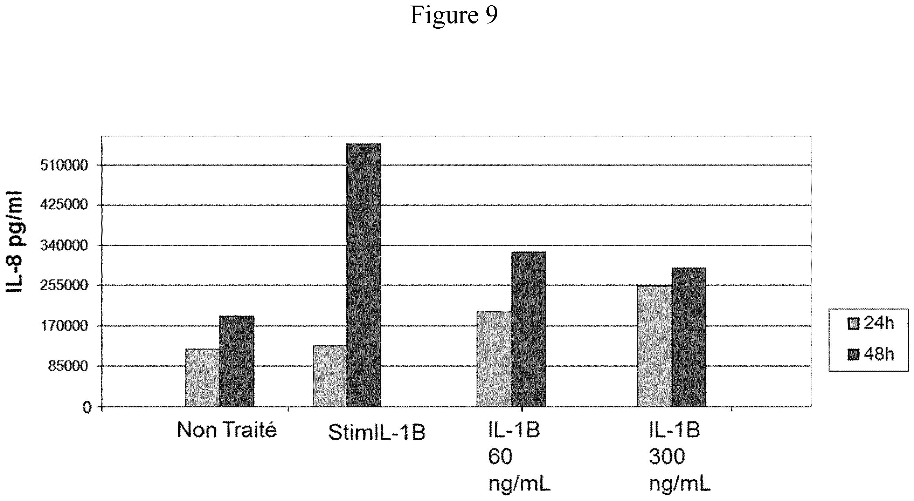

La **figure 9** représente la production de la cytokine IL-8 par des **explants cutanés** après différentes combinaisons stimulantes après 24 et 48 heures d'incubation. La combinaison Stim.IL-1B représente le réactif de stimulation contenant 60ng/mL d'IL-1β, 10µg/mL de Poly I:C, et 5µg/mL de Pam3CSK4. Une gamme de concentration avec le réactif IL-1β seul a été évaluée.

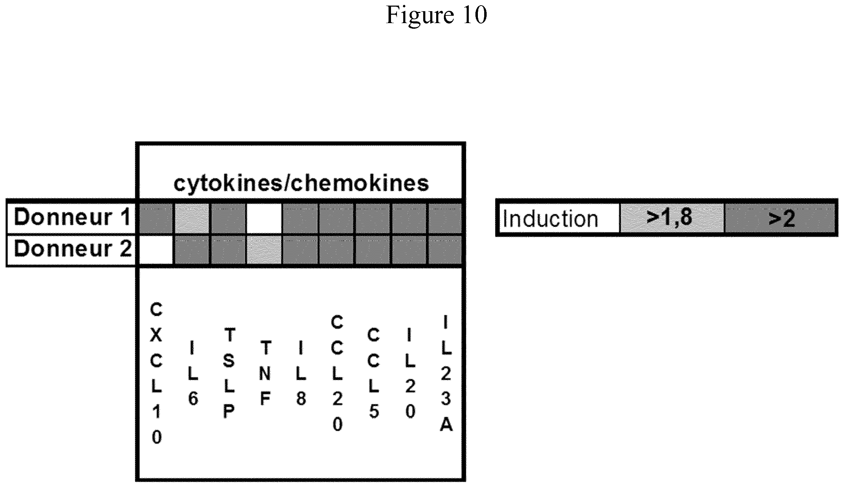

La **figure 10** représente l'expression de différents gènes codant pour des cytokines et chemokines (CXCL10, TSLP, IL-6, IL-8, IL-20, IL-23A et TNF-A) par l'analyse en PCR quantitative en temps réel des ARNm extraits des **explants cutanés** après 24 heures de stimulation avec 60ng/mL d'IL-1β, 10µg/mL de Poly I:C, et 5µg/mL de Pam3CSK4 (stimulation complète). L'analyse est réalisée sur 2 donneurs.

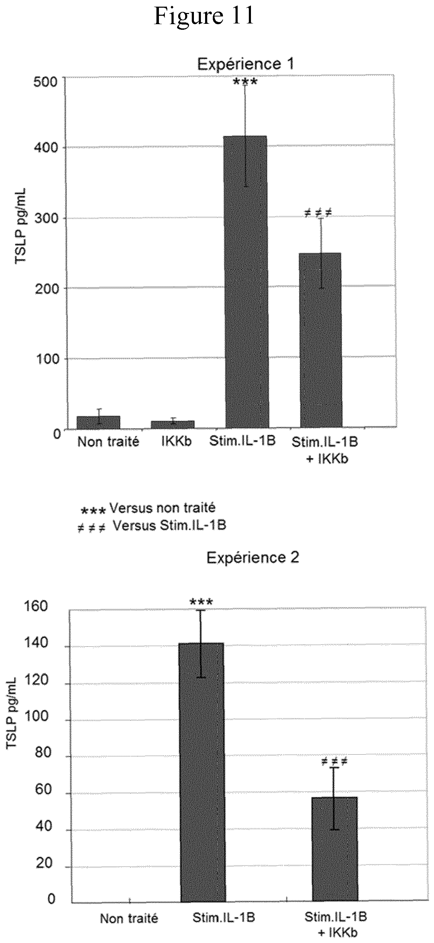

La **figure 11** représente la production de TSLP par des **épidermes reconstruits** après 24h de stimulation avec le réactif de stimulation (Stim.IL-1B) contenant 60ng/mL d'IL-1β, 10µg/mL de Poly I:C, et 5µg/mL de Pam3CSK4 en présence ou non un référent inhibiteur de NF-Kappaβ (10µM IKK Inhibitor X, Merck), (IKKb), sur 2 expériences indépendantes. ***: p<0.001 versus non traité, ^{≠≠≠}: p<0.001 versus Stim.IL-1B (test de Student).

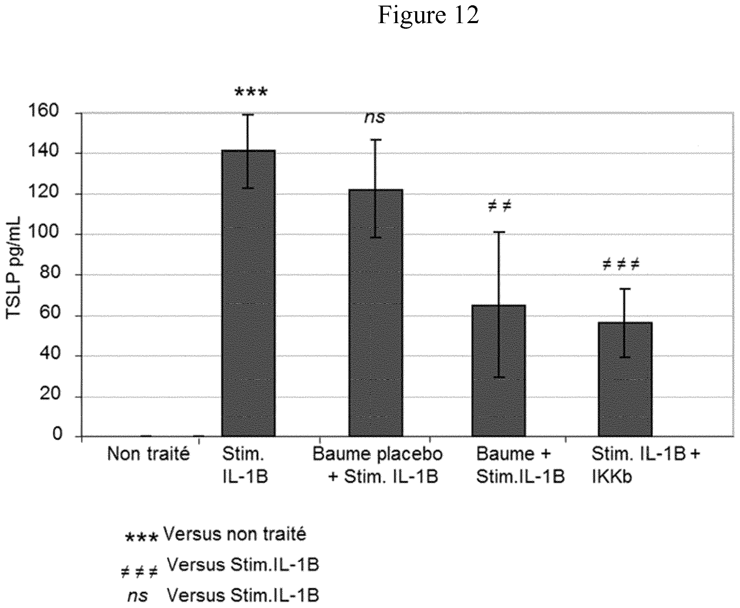

La **figure 12** représente la production de TSLP par des **épidermes reconstruits** traités par une application à la surface de 5mg/cm² du baume Exomega ou de la formulation excipiendaire correspondante (baume placebo) et après 24h de stimulation avec le réactif de stimulation (Stim.IL-1B) contenant 60ng/mL d'IL-1β, 10µg/mL de Poly I:C, et 5µg/mL de Pam3CSK4 KKb, ajout au réactif de stimulation d'un référent inhibiteur de NF-Kappaβ (10µM IKK Inhibitor X, Merck). ***: p<0.001 versus non traité, ^{≠≠≠}: p<0.001 versus Stim.IL-1B ^{≠≠}: p<0.01 versus Stim.IL-1B, (test de Student).

### Exemples

### I. CARACTERISATION DU MODELE TRIDIMENSIONNEL D'EPIDERMES RECONSTRUITS DE DERMATITE ATOPIQUE

### Génération d'épidermes reconstruits

Les cellules de la gaine externe de cheveux d'individu sain ont été isolées et mises en culture comme décrit dans la littérature (Use of Epidermal Equivalents Generated from Follicular Outer Root Sheath Cells in vitro and for Autologous Grafting of Chronic Wounds. Alain Limat, Thomas Hunziger. Cells Tissues Organs 2002;172:79-85) afin de produire des épidermes reconstruits (ER). A 14 jours de culture en interface air-liquide, les ER ont été stimulés avec différentes stimulations pro-inflammatoires.

### Caractérisation des stimulations pro-inflammatoires

La stimulation pro-inflammatoire a été testée en combinant un ou plusieurs des facteurs solubles suivants :
- IL-1β, et/ou
- Poly I:C, et/ou
- Pam3CSK4

Les concentrations de ces facteurs intégrées dans la combinaison de la stimulation pro-inflammatoire étaient les suivantes :
- 60ng/mL IL-1β
- 10µg/mL Poly I:C
- 5µg/mL Pam3CSK4

### Caractérisation du temps de stimulation

La durée de stimulation est comprise entre 4H et 24H.

### Traitements des épidermes

A la fin de la stimulation, les épidermes reconstruits ont été cryo-conservés pour des analyses histologiques et immunohistologiques ou bien lysés dans du tampon RLT (QIAGEN, Hilden, Germany) afin d'en extraire l'ARNm et procéder à une l'analyse transcriptionnelle. Les sous-nageants des épidermes ont été prélevés. Tous les échantillons ont été stockés à -80°C.

### Exemple 1: Analyse de TSLP et des autres marqueurs DA par dosage protéique (figures 1A, 2A, 3A, 4A et 5A)

### Matériel et méthodes

La concentration en TSLP des échantillons a été déterminée par test ELISA à l'aide d'un kit spécifique (DuoSet, R&D Systems). La production de RANTES/CCL5, MCP-3/CCL7, d'IL-6 et/ou d'IL-8 a été analysée grâce à la technologie Luminex® xMAP® selon les instructions du fournisseur (HCYTOMAG-60K, Millipore, Saint Charles, Missouri, USA). Les seuils minimum de détection pour TSLP, RANTES/CCL5, MCP-3/CCL7, IL-6 and IL-8 dans les sous-nageants de culture ont été de 15.6, 1.2, 3.8, 0.9 and 0.4 pg/mL respectivement. Tous les résultats en dessous de ces seuils ont été considérés comme nuls.

### Résultats

- La stimulation IL-1B permet une forte production de TSLP sécrétée dans les sous-nageants de culture des épidermes reconstruits (figure 1A).
- La stimulation IL-1B + Poly IC permet une forte production de TSLP sécrétée dans les sous-nageants de culture des épidermes reconstruits (figure 1A).
- La stimulation IL-1B + PAM3CSK4 permet une forte production de TSLP sécrétée dans les sous-nageants de culture des épidermes reconstruits (figure 1A).
- La stimulation IL-1B + Poly IC + PAM3CSK4 permet une forte production de TSLP sécrétée dans les sous-nageants de culture des épidermes reconstruits (figure 1A).
- La stimulation IL-1B additionnée de Poly IC ou PAM3CSK4 augmente de façon synergique la production de RANTES/CCL5 (figure 2A).
- La stimulation IL-1B + Poly IC + PAM3CSK4 permet la plus forte production de RANTES/CCL5 relarguée dans les sous-nageants de culture des épidermes reconstruits (figure 2A).
- La combinaison de deux des trois composants parmi IL-1B ou Poly IC ou PAM3CSK4 permet une sécrétion plus importante de la chemokine MCP3/CCL7 que lorsque la stimulation est composée d'un seul réactif (figure 3A).
- La stimulation IL-1B + Poly IC + PAM3CSK4 permet la plus forte production d'IL-6 sécrétée dans les sous-nageants de culture des épidermes reconstruits (figure 4A).
- La stimulation IL-1B + Poly IC + PAM3CSK4 permet la plus forte production d'IL-8 sécrétée dans les sous-nageants de culture des épidermes reconstruits (figure 5A).

### Exemple 2 : Profil d'expression génique (PCR quantitative en temps réel)

### Matériel et méthode

L'ARN total a été extrait des tissus reconstruits à l'aide du kit RNeasy® Fibrous Tissue Mini Kit (QIAGEN, Hilden, Germany) qui comporte une étape de DNAse et une étape de Protéinase K. Les extractions ont été réalisées à l'aide de l'appareil QIAcube (QIAGEN) selon les instructions du fournisseur. La concentration des ARN et leur qualité a été vérifiée grâce au spectrometreNanoDrop ND-1000 (Thermo Fisher Scientific Wilmington, Delaware, USA). Puis, 1µg d'ARN purifié a été rétrotranscrit grâce au mix High Capacity RNA-to-cDNA Master Mix (Life Technologies, Grand Island, New-York, USA). Les expériences Taqman ont ensuite été réalisées sur l'appareil ViiA™ 7 System Real-Time PCR System grâce à la technologie TaqMan® LowDensityArrays (Life Technologies, Grand Island, New-York). L'analyse des résultats a été faite avec le logiciel Data Assist (v3.0 Life Technologies, Grand Island, New-York, USA).

### Résultats

- Après 4h de stimulation avec IL-1B + Poly IC + PAM3CSK4, l'expression de TSLP en ARNm est fortement augmentée en comparaison du groupe témoin (figure 1B).
- L'expression génique de RANTES/CCL5, MCP-3/CCL7, IL-6 et IL-8 est augmentée 4h après la stimulation IL-1B + Poly IC + PAM3CSK4 en comparaison du groupe témoin, corroborant les résultats observés au niveau protéique par les dosages correspondants (figures 2B, 3B, 4B et 5B).

### Exemple 3 : Analyses de la filaggrine et de la claudine-1 après stimulation avec le milieu de maintenance contenant IL-1B + Poly IC + PAM3CSK4 (figure 6)

### Matériel et méthodes

Les coupes des tissus cryo-préservés ont une épaisseur de 5µm. Ces coupes ont été réalisées à l'aide d'un cryostat et colorées à l'Hémalin/Eosine.

Les marquages immuno-histologiques ont été réalisés avec les anticorps suivants : Filaggrine (FLG, 1:200, Vector, VP-F706 [Burlingame, California, USA]), Claudine 1 (CLD1, 1:500, DBS, RP 153-05 [Pleasanton, California, USA]). L'anticorps secondaire correspondant a ensuite été appliqué et le kit peroxydase system a été utilisé (Vectastain Elite ABC Universal kit, Vector [Burlingame, California, USA]) avec les réactifs du kit ImmPACT DAB Substrate (Vector [Burlingame, California, USA]). Plusieurs coupes de chaque spécimen ont été réalisées afin d'être représentatif.

La semi quantification des marquages de la filaggrine et de la claudine-1 a été réalisée avec un logiciel d'analyse d'images du type ImageJ (NIH) [au minimum 5 prises de vue par conditions]. Pour chaque prise de vue, le marquage a été déterminé comme un ratio de la zone marquée sur la surface épidermique totale.

### Résultats :

Au vu des résultats obtenus, la Demanderesse a mis en évidence les points suivants :
- La stimulation IL-1β + Poly IC + PAM3CSK4 provoque une diminution de marquage de la filaggrine dans la couche granuleuse des épidermes reconstruits en comparaison au témoin. La semi-quantification confirme que la réduction est significative et supérieure à 50%.
- L'expression génique de la filaggrine est réduite 24h après la stimulation IL-1β + Poly IC + PAM3CSK4 en comparaison du groupe témoin, corroborant les résultats obtenus avec les immuno-marquages de la protéine correspondante.
- La stimulation IL-1B + Poly IC + PAM3CSK4 provoque une diminution de marquage de la claudine-1 dans les couches supra-basales des épidermes reconstruits en comparaison au témoin. La semi-quantification confirme que la réduction est significative et de 60%.

### II - CARACTERISATION DU MODELE TRIDIMENSIONNEL D'EPIDERMES RECONSTRUITS ET D'EXPLANTS DE PEAU DE DERMATITE ATOPIQUE

### Exemple 4: Analyse de la sécrétion de TSLP et de IL-8 par dosage protéique (Figures 8, 9, 11)

### Génération d'épidermes reconstruits :

Les épidermes reconstruits ont été générés *in vitro* par la technique décrite dans Poumay Y, Dupont F, Marcoux S, Leclercq-Smekens M, Hérin M, Coquette A. Arch Dermatol Res. 2004 Oct;296(5):203-11.

### Matériel et méthodes

La sécrétion de TSLP a été déterminée par test ELISA à l'aide d'un kit spécifique (DuoSet, R&D Systems). La sécrétion d'IL-8 a été réalisée par le kit HTRF 62IL8PEB (Cisbio Bioassays). Sur explant de peaux, l'analyse statistique est faite sur la moyenne de TSLP sécrétée à partir des 3 donneurs (n=3) par comparaison multiple entre les groupes par la méthode d'ANOVA (one-way analysis of variance) avec le test de Dunnett' en utilisant Graph Pad Prism. Pour les épidermes reconstruits, l'analyse statistique est faite pour chaque expérience indépendante avec le test de Student non apparié (Student's t test) en utilisant Graph Pad Prism. La significativité est démontrée lorsque la p value <0.05.

### Résultats sur explants de peau humaine

- La stimulation IL-1β + Poly IC + PAM3CSK4 (Stim. IL-B), en particulier après 48h d'incubation, permet une forte production de TSLP sécrétée dans les sous-nageants de culture des explants cutanés (figure 8). Cette production est significative par rapport au témoin non traité. Une plus forte production est obtenue avec la concentration d'IL-1β à 300 ng/mL en particulier après 48h d'incubation.
- La stimulation IL-1β + Poly IC + PAM3CSK4 (Stim. IL-B), permet une forte production d'IL-8 sécrétée dans les sous-nageants de culture des explants cutanés (figure 9) après 48h d'incubation.

### Résultats sur épidermes reconstruits

- La stimulation IL-1β + Poly IC + PAM3CSK4 (Stim. IL-B), permet une forte production de TSLP sécrétée dans les sous-nageants de culture des épidermes reconstruits (figure 11). Cette production est significative par rapport au témoin non traité. L'addition du référent inhibiteur de NF-Kappaβ diminue de manière significative la production de TSLP.

### Exemple 5 : Profil d'expression génique (PCR quantitative en temps réel) (Figure 10)

### Matériel et méthode

L'ARN total a été extrait des explants cutanés par un broyage au préalable des tissus (Tissue Lyser) puis à l'aide du kit RNeasy® Fibrous Tissue Mini Kit (QIAGEN, Hilden, Germany). Les extractions ont été réalisées à l'aide de l'appareil QIAcube (QIAGEN) selon les instructions du fournisseur. La concentration des ARN et leur qualité a été vérifiée grâce au spectrometreNanoDrop ND-1000 (Thermo Fisher Scientific Wilmington, Delaware, USA). Puis, 1µg d'ARN purifié a été rétrotranscrit grâce au mix High Capacity RNA-to-cDNA Master Mix (Life Technologies, Grand Island, New-York, USA). Les expériences Taqman ont ensuite été réalisées sur l'appareil ViiA™ 7 System Real-Time PCR System grâce à la technologie TaqMan® LowDensityArrays (Life Technologies, Grand Island, New-York). L'analyse est réalisée à partir de réplicats d'explants et sur 2 donneurs.

### Résultats

La stimulation complète (iL-1β + Poly IC + PAM3CSK4) induit une induction de plusieurs gènes codant pour des cytokines et chemokines en comparaison du groupe témoin non stimulé (Figure 10). En particulier, la stimulation entraine l'induction de l'expression des cytokines TSLP et IL-8, ce qui corrobore les résultats obtenus en dosage protéique (figure 8 et 9).

### III. EVALUATION DE PRODUIT FINI dans l'indication peaux très sèches, atopiques : Mise en évidence de l'efficacité de la crème Exomega

### Exemple 6: Mise en évidence de l'efficacité de la crème Exomega sur des épidermes reconstruits selon l'invention

### Matériel et méthodes

Des épidermes reconstruits générés comme décrits dans l'exemple 3 ont été traités avec une crème placebo ou la crème A-Derma Exomega DEFI. Les crèmes ont été appliquées à la surface du stratum cornéum des épidermes. Les épidermes ont ensuite été incubés dans du milieu de culture supplémenté avec la stimulation telle que décrite contenant IL1β, Poly :IC et PAM3CSK4. A la fin de l'incubation, les surnageants des épidermes ont été récoltés pour effectuer un dosage TSLP par un test ELISA.

### Résultats

Les résultats sont présentés sur la figure 7.

La crème Placebo n'inhibe pas dans ces conditions la production de TSLP; La crème A-Derma Exomega DEFI inhibe significativement la production de TSLP. Les moyennes représentent les valeurs de n= 2 donneurs en triplicats d'épidermes et duplicats techniques. (soit au total n = 12 valeurs)

### Exemple 7 : Effet de l'application d'une crème à indication dermatite atopique sur des épidermes reconstruits générés in vitro par la technique de Poumay et al.

### Materiel et méthodes

Des épidermes reconstruits ont été générés comme décrits dans Poumay Y, Dupont F, Marcoux S, Leclercq-Smekens M, Hérin M, Coquette A. Arch Dermatol Res. 2004 Oct;296(5):203-11. Ces épidermes ont ensuite été traités avec une crème placebo ou le baume A-Derma Exomega. Les formulations ont été appliquées à la surface du stratum corneum des épidermes. Ils ont été stimulés par le réactif pro-inflammatoire, contenant 60ng/mL d'IL-1β, 10µg/mL de Poly I:C, et 5µg/mL de Pam3CSK4 (Figure 12).

### Résultats

L'application du baume A-Derma Exomega à la surface des épidermes réalisée avant la stimulation IL-1β + Poly IC + PAM3CSK4 (Baume + Stim. IL-B), permet une diminution significative de TSLP sécrétée dans les sous-nageants de culture des épidermes reconstruits (figure 12). Cette diminution n'est pas observée lorsque les épidermes reconstruits ont été traités avec la formulation excipiendaire du baume (Baume placebo + Stim. IL-B).

## Revendications

1. Utilisation d'un réactif de stimulation pro-inflammatoire contenant entre 10 et 500ng/mL d'interleukine IL-1β pour reproduire *in vitro* la physiopathologie de la dermatite atopique et/ou altérer la fonction barrière dans une peau reconstruite ou un épiderme reconstruit qui contient des cellules différenciées réparties en plusieurs couches, ou dans un explant cutané, ledit réactif de stimulation pro-inflammatoire comprenant en outre un ligand du récepteur TLR-2 choisi parmi un peptidoglycane, de l'acide lipoteichoique, une lipoprotéine, un lipoarabinomanne, et du zymosan, et/ou un ligand du récepteur TLR-3 choisi parmi le poly (A :U) et le poly I:C.

2. Utilisation selon la revendication 1, **caractérisée en ce que** ledit ligand du récepteur TLR-2 est Pam3 CSK4 et **en ce que** ledit ligand du récepteur TLR-3 est le poly I:C.

3. Utilisation selon l'une quelconque des revendications 1 à 2, **caractérisée en ce que** ledit réactif de stimulation pro-inflammatoire contient :
a. Entre 10 et 500 ng/mL d'interleukine IL-1β, et
b. Entre 3 et 20 µg/mL d'un ligand du récepteur TLR-2, et/ou
c. Entre 3 et 20 µg/mL d'un ligand du récepteur TLR-3.

4. Procédé pour reproduire *in vitro* la physiopathologie de la dermatite atopique et/ou altérer la fonction barrière dans une peau reconstruite ou un épiderme reconstruit qui contient des cellules différenciées réparties en plusieurs couches, ou dans un explant cutané, ladite méthode contenant les étapes :
a. Mettre en contact ladite peau reconstruite, ledit épiderme reconstruit, ou ledit explant cutané avec un milieu de maintenance contenant un réactif de stimulation pro-inflammatoire contenant entre 10 et 500ng/mL d'interleukine IL-1β, ledit milieu de maintenance contenant en outre entre 3 et 20 µg/mL d'un ligand du récepteur TLR-2, et/ou entre 3 et 20 µg/mL d'un ligand du récepteur TLR-3, et
b. Incuber pendant 2 à 48 heures.

5. Peau reconstruite ou épiderme reconstruit qui contient des cellules différenciées réparties en plusieurs couches, ou explant cutané reproduisant la physiopathologie de la dermatite atopique, obtenu par le procédé de la revendication 4, ladite peau reconstruite ou ledit épiderme reconstruit sécrétant la cytokine TSLP.

6. Méthode de criblage pour l'identification *in vitro* de composés destiné au traitement de la dermatite atopique et des peaux sèches à tendance atopique, comprenant la mise en contact, par application topique ou par ajout dans le milieu de culture, desdits composés avec la peau reconstruite, l'épiderme reconstruit ou l' explant cutané tel que définis dans la revendication 5, et comprenant les étapes :
a. Mesurer la quantité de TSLP sécrétée,
b. Comparer la valeur obtenue à l'étape a) avec celle mesurée en l'absence du composé candidat.

## Patentansprüche

1. Verwendung eines pro-inflammatorischen Stimulationsreagens, enthaltend zwischen 10 und 500 ng/mL Interleukin IL-1β für die Reproduktion *in vitro* der Physiopathologie der atopischen Dematitis und/oder Beeinträchtigung der Barrierefunktion in einer rekonstruierten Haut oder einer rekonstruierten Epidermis, die differenzierte Zellen enthält, die in mehreren Schichten verteilt sind, oder in einem Hautexplantat, wobei das pro-inflammatorische Stimulationsreagens ferner einen Liganden des Rezeptors TLR-2, ausgewählt aus einem Peptidoglykan, Lipoteichonsäure, einem Lipoprotein, einem Lipoarabinoman und Zymosan, und/oder einen Liganden des Rezeptors TLR-3, ausgewählt aus dem Poly(A:U) und dem Poly I:C, umfasst.

2. Verwendung nach Anspruch 1, **dadurch gekennzeichnet, dass** der Ligand des Rezeptors TLR-2 Pam3CSK4 ist und dass der Ligand des Rezeptors TLR-3 das Poly I:C ist.

3. Verwendung nach einem der Ansprüche 1 bis 2, **dadurch gekennzeichnet, dass** das pro-inflammatorische Stimulationsreagens enthält:
a. zwischen 10 und 500 ng/mL Interleukin IL-1β, und
b. zwischen 3 und 20 µg/mL eines Liganden des Rezeptors TLR-2, und/oder
c. zwischen 3 und 20 µg/mL eines Liganden des Rezeptors TLR-3.

4. Verfahren für die Reproduktion *in vitro* der Physiopathologie der atopischen Dermatitis und/oder Beeinträchtigung der Barrierefunktion in einer rekonstruierten Haut oder einer rekonstruierten Epidermis, die differenzierte Zellen enthält, die in mehreren Schichten verteilt sind, oder in einem Hautexplantat, wobei die Methode die folgenden Schritte enthält:
a. Inkontaktversetzen der rekonstruierten Haut, der rekonstruierten Epidermis oder des Hautexplantats mit einem Instandhaltungsmilieu, das ein proinflammatorisches Stimulationsreagens enthält, enthaltend zwischen 10 und 500 ng/mL Interleukin IL-1β, wobei das Instandhaltungsmilieu ferner zwischen 3 und 20 µg/mL eines Liganden des Rezeptors TLR-2 und/oder zwischen 3 und 20 µg/mL eines Liganden des Rezeptors TLR-3 enthält, und
b. Inkubieren während 2 bis 48 Stunden.

5. Rekonstruierte Haut oder rekonstruierte Epidermis, die differenzierte Zellen enthält, die in mehreren Schichten verteilt sind, oder Hautexplantat, das die Physiopathologie der atopischen Dermatitis reproduziert, erhalten durch das Verfahren von Anspruch 4, wobei die rekonstruierte Haut oder die rekonstruierte Epidermis das Zytonkin TSLP sekretiert.

6. Screening-Methode für die Identifizierung *in vitro* von Verbindungen, die zur Behandlung der atopischen Dermatitis und der trockenen Haut mit atopischer Tendenz bestimmt sind, umfassend das Inkontaktversetzen, durch topisches Auftragen oder durch Hinzufügen in das Kulturmilieu, der Verbindungen mit der rekonstruierten Haut, der rekonstruierten Epidermis oder dem Hautexplantat nach Anspruch 5, und umfassend die folgenden Schritte:
a. Messen der sekretierten TSLP-Menge,
b. Vergleichen des erhaltenen Werts in Schritt a) mit dem, der bei Abwesenheit der Kandidatenverbindung gemessen wurde.

## Claims

1. Use of a pro-inflammatory stimulation reagent containing between 10 and 500 ng/mL interleukin IL-1β for reproducing *in vitro* the pathophysiology of atopic dermatitis and/or altering the barrier function in reconstructed skin or reconstructed epidermis which contains differentiated cells distributed in several layers, or in a skin explant, said pro-inflammatory stimulation reagent further comprising a TLR-2 receptor ligand selected from a peptidoglycan, lipoteichoic acid, a lipoprotein, a lipoarabinomannan, and zymosan, and/or a TLR-3 receptor ligand selected from poly (A:U) and poly I:C.

2. Use as claimed in claim 1, **characterized in that** said TLR-2 receptor ligand is Pam3CSK4 and **in that** said TLR-3 receptor ligand is poly I:C.

3. Use as claimed in any one of claims 1 to 2, **characterized in that** said pro-inflammatory stimulation reagent contains:
a. Between 10 and 500 ng/mL interleukin IL-1β, and
b. Between 3 and 20 µg/mL TLR-2 receptor ligand, and/or
c. Between 3 and 20 µg/mL TLR-3 receptor ligand.

4. A process for reproducing *in vitro* the pathophysiology of atopic dermatitis and/or altering the barrier function in reconstructed skin or reconstructed epidermis which contains differentiated cells distributed in several layers, or in a skin explant, said method containing the steps:
a. Bringing said reconstructed skin, said reconstructed epidermis, or said skin explant into contact with a maintenance medium containing a pro-inflammatory stimulation reagent containing between 10 and 500 ng/mL interleukin IL-1β, said maintenance medium further containing between 3 and 20 µg/mL TLR-2 receptor ligand, and/or between 3 and 20 µg/mL TLR-3 receptor ligand, and
b. Incubating for 2 to 48 hours.

5. Reconstructed skin or reconstructed epidermis which contains differentiated cells distributed in several layers, or skin explant reproducing the pathophysiology of atopic dermatitis, obtained by the process as claimed in claim 4, said reconstructed skin or reconstructed epidermis secreting the cytokine TSLP.

6. A screening method for *in vitro* identification of compounds intended for the treatment of atopic dermatitis and of dry skin with atopic tendency, comprising bringing said compounds into contact, by topical application or by addition to the culture medium, with the reconstructed skin, reconstructed epidermis or skin explant as defined in claim 5, and comprising the steps:
a. Measuring the amount of TSLP secreted,
b. Comparing the value obtained in step a) with that measured in the absence of the candidate compound.
